# EUROPEAN PATENT SPECIFICATION

(11) **EP 3 236 943 B1**
(45) Date of publication and mention of the grant of the patent: **25.01.2023**
(21) Application number: 15834774.0
(22) Date of filing: 23.12.2015
(51) Int. Cl.: A61K 9/00, A61K 9/28, A61K 9/50, A61K 31/519, A61P 35/00, A61P 35/02

(54) **COMPOSITIONS FOR ILEO-JEJUNAL DRUG DELIVERY**
ZUSAMMENSETZUNGEN ZUR ILEO-JEJUNALEN ARZNEIMITTELABGABE
COMPOSITIONS POUR ADMINISTRATION DE MÉDICAMENTS ILÉO-JÉJUNAL.

(30) Priority: 24.12.2014 US 201462096812 P
(43) Date of publication of application: 01.11.2017
(73) Proprietor: Principia Biopharma Inc., S. San Francisco, CA 94080 (US)
(72) Inventor: NUNN, Philip, A., Mountain View CA 94041 (US); BERNER, Bret, Half Moon Bay CA 94019 (US); MASJEDIZADEH, Mohammad, Reza, San Jose CA 95124 (US)
(74) Representative: J A Kemp LLP
(86) International application number: PCT/US2015/000515
(87) International publication number: WO 2016/105582

(56) References cited:
- WO-A1-2013/191965
- WO-A1-2014/004707
- WO-A1-2014/039899

## Description

### CROSS-REFERENCE

This application claims the benefit of priority to United States provisional patent application serial number 62/096,812, filed December 24, 2014.

### FIELD

The present disclosure provides oral pharmaceutical dosage forms comprising a reversible or irreversible covalent drug molecules that do not release the active ingredient in the stomach and substantially release the drug molecule in the ileum and jejunum of the small intestine and use of these formulations for the treatment of diseases treatable by such compounds such as cancer and autoimmune diseases.

### BACKGROUND

Targeted therapy has received increased attention particularly in the oncology area due to the clinical success of kinase inhibitors as anti-cancer agents. The ongoing challenges to the development of targeted therapies include achieving high selectivity for the primary target and prolonged inhibition to maximize their therapeutic efficacy. Covalent drugs have become a highly attractive approach to designing next generation targeted therapies due to their enhanced ability to achieve high selectivity as well as prolonged inhibition even with significantly reduced systemic exposure of the drugs. Covalent drugs achieve their high selectivity and exceptional potency due to the covalent interaction with a specific cysteine residue in the active site of proteins for which the drug molecule binds. This covalent binding additionally provides prolonged efficacy with increased duration of action that outlasts the systemic exposure of the drug. Drugs containing an acrylamide moiety as a Michael acceptors generally react irreversibly with thiols like glutathione and may also react irreversibly with proteins other than the desired target, especially proteins with hyper-reactive cysteines.

Reversible covalent drug molecules *i.e*., drugs which contain a Michael acceptor with a second electron withdrawing group, can exhibit poor bioavailability or delayed for systemic absorption when the drug is administered orally which can be manifested by low plasma AUC and/or Cₘₐₓ values resulting in suboptimal efficacy *in vivo.* The poor bioavailability of this new class of drugs can be attributed, in part, to the reactivity of reversible covalent Michael acceptors moiety in these drugs. Accordingly, limiting the exposure of the reversible covalent drugs to the stomach where the combination of low pH and digestive or metabolic enzymes and other sources of thiols occur, a significant increase in systemic exposure of the drug can be obtained.

In addition, limiting the exposure of irreversible covalent drug molecules to the stomach may also lead to a significant increase in systemic exposure of the drug and a reduction in potential adverse side effects such as diarrhea, nausea or emesis, and dizziness. For example, when ibrutinib, an irreversible covalently bound drug molecule, is administered intraduodenally, the bioavailability increased unexpectedly from 21 % to 100% compared to direct oral administration as determined by AUC. (D. M. Goldstein, Formulations Comprising Ibrutinib, WO 2014/004707 published 3 January 2014) Gastric bypass of ibrutinib should increase bioavailability and/or reduce or altogether eliminate potential adverse side effects of this drug such as diarrhea, nausea or emesis, and dizziness.

Furthermore, the expression of metabolizing enzymes, such as cysteine proteases, mucins, transporters and reactive thiol containing molecules in the stomach, such as glutathione, can also contribute to the low oral bioavailability of reversible and irreversible covalent Michael acceptor- containing drugs (*see*, *e*.*g*., Johnson D. S., et. al., Future Med Chem. 2010 June 1; 2(6):949-964 and Potashman M. H. et al. J. Med. Chem., Vol 52, No. 5. Pgs. 1231-1246). For example, the combination of digestive enzymes, such as the cysteine protease, pepsin, transporters and metabolizing enzymes such as CYP enzymes in the gastric mucosa, can result at low pH in high chemical and/or metabolic transformation of the reversible and irreversible covalent Michael acceptors. Accordingly, by avoiding exposure of the reversible and irreversible covalent drugs to the stomach where the combination of low pH and digestive or metabolic enzymes and other sources of thiols occur, a significant increase in systemic exposure of these drugs can be obtained. Additionally, avoidance of exposure to the stomach may reduce or altogether eliminate potential adverse side effects of these drugs such as diahrrea and emesis, commonly called vomiting.

WO 2013/191965 describes oral pharmaceutical formulations comprising reversible covalent compounds having a Michael acceptor moiety. WO 2014/039899 describes reversible covalent inhibitors of tyrosine kinases, in particular BTK.

### SUMMARY

It has now been shown that there is an unexpected increase in absorption of drug molecules disclosed herein in the jejunum and ileum compared to the duodenum and bioavailability can be further improved with dosage forms that release the drug molecule primarily distal to the duodenum and optimally in the ileum and jejunum. This result is surprising since release of the drug further along in the gastrointestinal tract reduces the path length for drug absorption and would be expected to reduce bioavailability.

The present invention provides a solid oral dosage form, which is a tablet or capsule, comprising:
(A) a core comprising a compound chosen from: 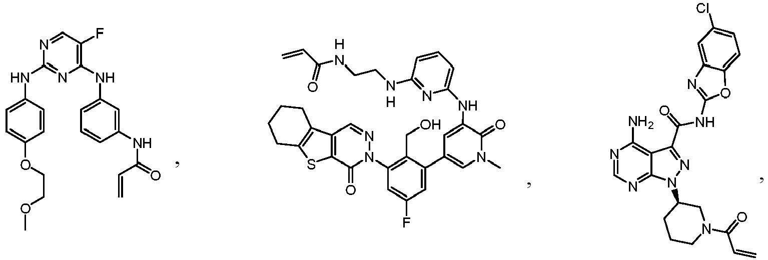 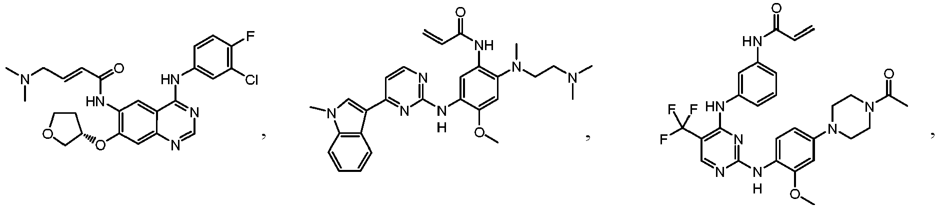 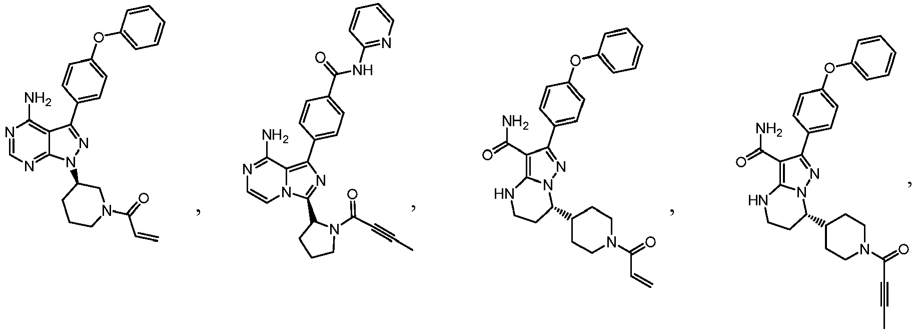 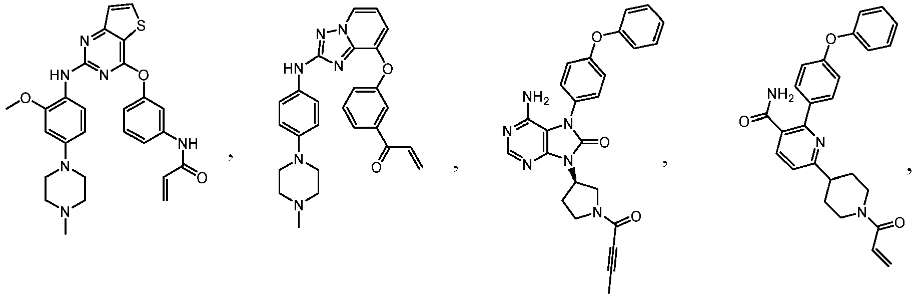 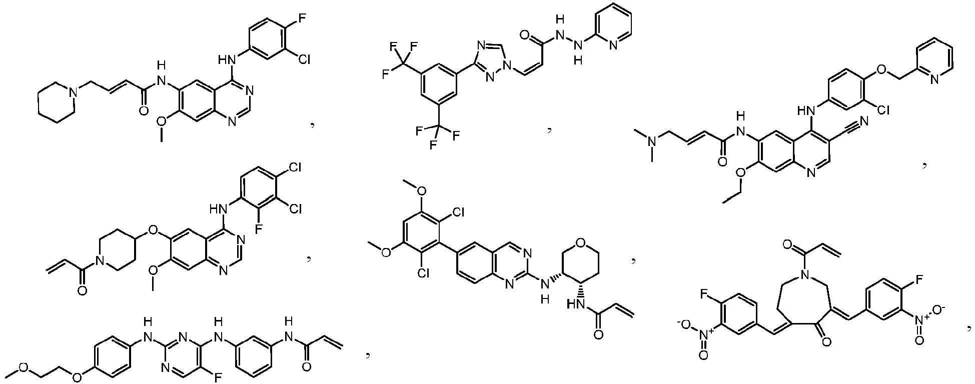 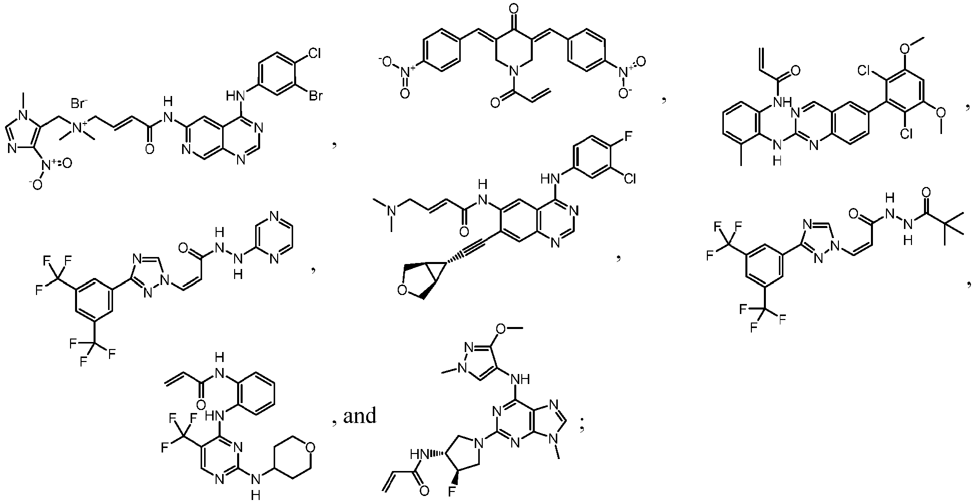 and/or a pharmaceutically acceptable salt thereof;
(B) an enteric coating;
(C) a subcoat between the enteric coating and the core, wherein the subcoat is:
   (1) a water soluble or hydrophilic erodible polymer, said polymer being a low molecular weight polymer selected from hydroxymethyl cellulose (HPMC), hydroxyethyl cellulose, hydroxymethyl cellulose, hydroxypropyl cellulose, microcrystalline cellulose, polyvinylpyrrolidiones, polysaccharides (or a polysaccharide derivative), polyvinyl alcohols, polyethylene glycol (PEG), polypropylene glycol (PPG), and PEG-PPG block copolymers; or
   (2) a water insoluble composition comprising: (i) particles of a water soluble compound capable of forming channels in the water insoluble composition; or (ii) water insoluble hydrophilic particles which cause swelling of the subcoat when in contact with an aqueous media; and
(D) a pharmaceutically acceptable excipient,
wherein the solid oral dosage form has an onset of release of the compound and/or said pharmaceutically acceptable salt thereof of the core (A) into the ileo-jejunal portion of the small intestine of a mammal after oral ingestion of the dosage form by the mammal; and
wherein the solid oral dosage form is able to release at least 80% by weight of said compound and/or said pharmaceutically acceptable salt thereof in 120 minutes in a dissolution vessel comprising an aqueous solution at a pH of from 6.4 to 7.4.

The solid oral dosage form may release a drug molecule (i.e., the compound and/or pharmaceutically acceptable salt thereof of the core (A)) at a predetermined locus in the intestine after oral ingestion of the dosage form by a mammal.

The solid oral dosage form delivers a drug molecule to the ileo-jejunal portion of the small intestine of a mammal.

In one aspect, the solid oral dosage form is a delayed release solid oral dosage form that releases a drug molecule when the dosage form encounters a specific pH in the gastrointestinal tract of a mammal.

The solid oral dosage form comprises at least one enteric coating covering the core which enteric coat remains intact in the stomach of a mammal.

The solid oral dosage form is able to release the drug molecule in the ileum and/or jejunum of a mammal resulting in enhanced bioavailability of the drug molecule as compared to immediate release of the drug molecule after ingestion.

In another aspect, the solid oral dosage form releases the drug molecule as a bolus in the ileum and/or jejunum of the small intestine of a mammal resulting in enhanced bioavailability of the drug molecule as compared to immediate release of the drug molecule after ingestion.

The solid oral dosage form comprises a core containing a drug molecule in combination with additional subcoating(s), diluents or excipients designed to substantially release the drug molecule in the ileum and/or jejunum of the small intestine of a mammal.

In another aspect, the solid oral dosage form comprises a core covered by a water insoluble subcoat capable of swelling or forming channels allowing influx of water into the core which results in swelling of the core, rupturing the subcoat, and release of the contents of the core as a bolus in the intestine.

In another aspect, the solid oral dosage form contains a core covered by a subcoat capable of forming channels which cause influx of water into the core and subsequent efflux of the drug molecule through the channels as the core passes through the intestine.

In another aspect, the solid oral dosage form contains a core covered by a water soluble polymer which dissolves at a predetermined pH in the intestine.

The invention further provides a solid oral dosage form as defined herein for use in a method of treating a disease treatable by inhibition of BTK, in a patient in recognized need thereof, wherein said method comprises administering to the patient a therapeutically effective amount of the compound and/or the pharmaceutically acceptable salt thereof of the core (A) in single or multiple doses. Said solid oral dosage form typically has improved bioavailability compared to formulations which result in immediate release of the drug molecule after ingestion.

### BRIEF DESCRIPTION OF THE FIGURES

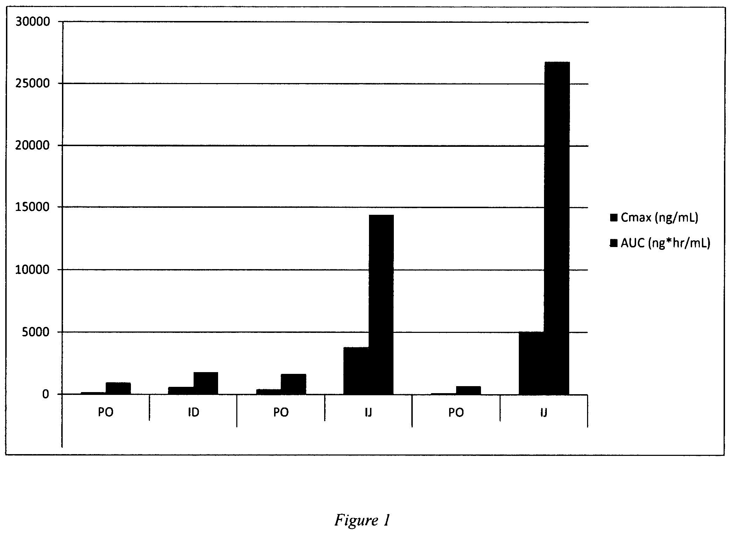

Figure 1 depicts the results of a pharmacokinetic experiment comparing oral and intra-duodenal gavage dosing of (R)-2-(3-(4-amino-3-(2-fluoro-4-phenoxyphenyl)-1H-pyrazolo[3,4-d]pyrimidin-1-yl)piperidine-1-carbonyl)-4-methyl-4-(4-(oxetan-3-yl)piperazin-1-yl)pent-2-enenitrile having E/Z ratio of about 9:1. The area under the curve (AUC) and the Cₘₐₓ are plotted.

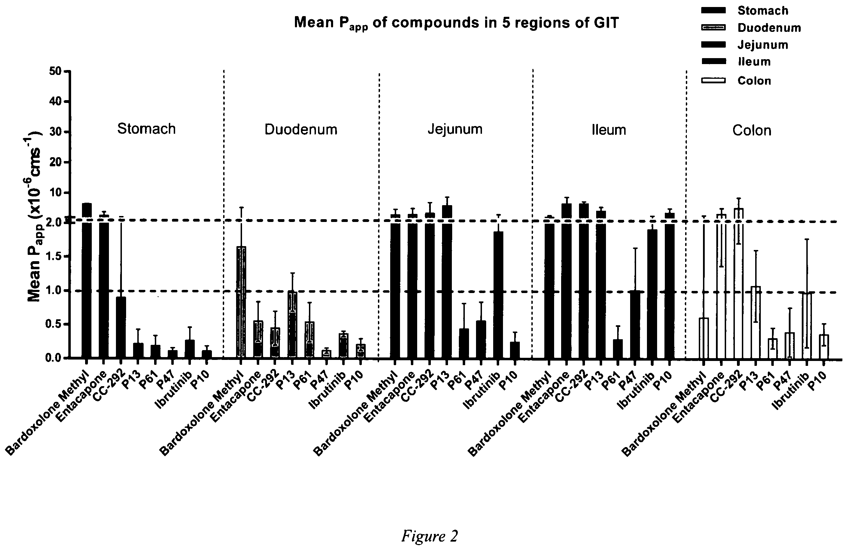

Figure 2 depicts the results of a study of the permeablilty of pharmacologically active compounds which are Michael reaction acceptors in various regions of the GI tract including the stomach, duodenum, ileum, jejunum and colon. Drugs studied include (R)-2-(3-(4-amino-3-(2-fluoro-4-phenoxyphenyl)-1H-pyrazolo[3,4-d]pyrimidin-1-yl)piperidine-1-carbonyl)-4-methyl-4-(4-(oxetan-3-yl)piperazin-1-yl)pent-2-enenitrile having E/Z ratio of about 9:1(P10), (R,E)-2-(3-(4-amino-3-(2-fluoro-4-phenoxyphenyl)-1H-pyrazolo[3,4-d]pyrimidin-1-yl)piperidine-1-carbonyl)-4,4-dimethylpent-2-enenitrile(P47), (R,E)-2-(3-(4-amino-3-(2-fluoro-4-phenoxyphenyl)-1H-pyrazolo[3,4-d]pyrimidin-1-yl)piperidine-1-carbonyl)-4-methyl-4-(4-methylpiperazin-1-yl)pent-2-enenitrile(P61), 8-(3-(4-acryloylpiperazin-1-yl)propyl)-6-(2,6-dichloro-3,5-dimethoxyphenyl)-2-(dimethylamino)pyrido[2,3-d]pyrimidin-7(8H)-one(P13), 1-[(3*R*)-3-[4-Amino-3-(4-phenoxyphenyl)-1*H*-pyrazolo[3,4-d]pyrimidin-1-yl]piperidin-1-yl]prop-2-en-1-one (ibrutinib), N-(3-((5-fluoro-2-((4-(2-methoxyethoxy)phenyl)amino)pyrimidin-4-yl)amino)phenyl)acrylamide (CC-292), (2E)-2-cyano-3-(3,4-dihydroxy-5-nitrophenyl)-N,N-diethylprop-2-enamide (entacapone), and methyl 2-cyano-3,12-dioxooleana-1,9(11)dien-28-oate (bardoxolone methyl).

### DETAILED DESCRIPTION

The indefinite article "a" or "an" entity as used herein refers to one or more of that entity; for example, a compound refers to one or more compounds or at least one compound unless stated otherwise. As such, the terms "a" (or "an"), "one or more", and "at least one" can be used interchangeably herein.

The term "independently" is used herein to indicate that a variable is applied in any one instance without regard to the presence or absence of a variable having that same or a different definition within the same compound. Thus, in a compound in which R" appears twice and is defined as "independently carbon or nitrogen", both R"s can be carbon, both R"s can be nitrogen, or one R" can be carbon and the other nitrogen.

When any variable (*e.g*., R¹, R^{4a}, Ar, X¹ or Het) occurs more than one time in any moiety or formula depicting and describing compounds employed or claimed in the present invention, its definition on each occurrence is independent of its definition at every other occurrence. Also, combinations of substituents and/or variables are permissible only if such compounds result in stable compounds.

A line drawn through a bond, " 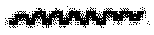 ", refers to the point of attachment of a functional group or other chemical moiety to the rest of the molecule of which it is a part. Thus, for example:

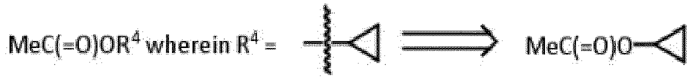

A bond drawn into ring system (as opposed to connected at a distinct vertex) indicates that the bond may be attached to any of the suitable ring atoms.

The term "optional" or "optionally" as used herein means that a subsequently described event or circumstance may, but need not, occur, and that the description includes instances where the event or circumstance occurs and instances in which it does not. For example, "optionally substituted" means that the optionally substituted moiety may incorporate a hydrogen or a substituent.

The term "about" is used herein to mean approximately, in the region of, roughly, or around. When the term "about" is used in conjunction with a numerical range, it modifies that range by extending the boundaries above and below the numerical values set forth. In general, the term "about" is used herein to modify a numerical value above and below the stated value by a variance of 5%.

As used herein, the recitation of a numerical range for a variable is intended to convey that the invention may be practiced with the variable equal to any of the values within that range. Thus, for a variable which is inherently discrete, the variable can be equal to any integer value of the numerical range, including the end-points of the range. Similarly, for a variable which is inherently continuous, the variable can be equal to any real value of the numerical range, including the end-points of the range. As an example, a variable which is described as having values between 0 and 2, can be 0, 1 or 2 for variables which are inherently discrete, and can be 0.0, 0.1, 0.01, 0.001, or any other real value for variables which are inherently continuous.

One aspect of this disclosure is directed to a solid oral dosage form for use in a method of treating a disease treatable by inhibition of a tyrosine kinase such as BLK, BMX, EGFR, HER2, HER4, ITK, TEC, BTK, and TXK in a patient which method comprises administering to the patient in recognized need thereof, a solid oral pharmaceutical formulation disclosed herein. In one embodiment, the tyrosine kinase is BTK.

BTK (UniProt accession number Q06187) is a member of the Tec family of tyrosine kinases, and has been shown to be a critical regulator of early B-cell development and mature B-cell activation and survival (Khan et al. Immunity 1995 3:283; Ellmeier et al. J. Exp. Med. 2000 192:1611).

For avoidance of doubt, the term "drug molecule" or "BTK inhibitor" as used herein refers to either the free base of a compound present in the core (A) and/or to a pharmaceutically acceptable salt of a compound present in the core (A) unless explicitly limited to the free base or a salt form.

In an embodiment, the drug molecule is a reversible or irreversible covalent kinase inhibitor. For example, in one non-limiting embodiment, a reversible or irreversible covalent kinase inhibitor is a BTK inhibitor.

In an embodiment, the drug molecule is a reversible covalent BTK inhibitor. The use of covalent modification to modulate drug targets has been reviewed. (I. M. Serfimova et al., Nature Chem. Biol. 2012 8:471; R. Mah et al. Bioorg. Med. Chem. Lett. 2014 24:33-39; J. Sing, et al., Nat. Rev. Drug Discov. 2011 10:307).

In yet another embodiment, the drug molecule is an irreversible covalent BTK inhibitor.

In another embodiment, the drug molecule is a reversible or irreversible covalent kinase inhibitor chosen from ibrutinib, ACP196, acalabrutinib, BGB3111, ONO-4059, CC-292, X-022, afatinib, mereletinib, osimertinib, rociletinib, neratinib, dacomitinib, poziotinib, spebrutinib, tarloxotinib, selinexor, verdinexor, PF-06747775, BLU-554, NSC-687852, VLX-1570, NT-113, BLU-9931, KPT-350, and AZ-13767370.

In another embodiment, the drug molecule is a reversible or irreversible covalent kinase inhibitor chosen from:
(R)-1-(3-(4-amino-3-(4-phenoxyphenyl)-1H-pyrazolo[3,4-d]pyrimidin-1-yl)piperidin-1-yl)prop-2-en-1 -one,
(R)-4-(8-amino-3-(1-(but-2-ynoyl)pyrrolidin-2-yl)imidazo[1,5-a]pyrazin-1-yl)-N-(pyridin-2-yl)benzamide,
(S)-7-(1-acryloylpiperidin-4-yl)-2-(4-phenoxyphenyl)-4,5,6,7-tetrahydropyrazolo[1,5-a]pyrimidine-3-carboxamide,
(S)-7-(1-(but-2-ynoyl)piperidin-4-yl)-2-(4-phenoxyphenyl)-4, 5,6,7-tetrahydropyrazolo[1,5-a]pyrimidine-3-carboxamide,
N-(3-((2-((2-methoxy-4-(4-methylpiperazin-1-yl)phenyl)amino)thieno[3,2-d]pyrimidin-4-yl)oxy)phenyl)acrylamide,
1-(3-((2-((4-(4-methylpiperazin-1-yl)phenyl)amino)-[1,2,4]triazolo[1,5-a]pyridin-8-yl)oxy)phenyl)prop-2-en-1 -one,
(R)-6-amino-9-(1-(but-2-ynoyl)pyrrolidin-3-yl)-7-(4-phenoxyphenyl)-7H-purin-8(9H)-one,
N-(2-((6-((5-(5-fluoro-2-(hydroxymethyl)-3-(4-oxo-6,7,8,9-tetrahydrobenzo[4,5]thieno[2,3-d]pyridazin-3(4H)-yl)phenyl)-1-methyl-2-oxo-1,2-dihydropyridin-3-yl)amino)pyridin-2-yl)amino)ethyl)acrylamide,
N-(3-((5-fluoro-2-((4-(2-methoxyethoxy)phenyl)amino)pyrimidin-4-yl)amino)phenyl)acrylamide,
6-(1-acryloylpiperidin-4-yl)-2-(4-phenoxyphenyl)nicotinamide,
(R)-1-(1-acryloylpiperidin-3-yl)-4-amino-N-(5-chlorobenzo[d]oxazol-2-yl)-1H-pyrazolo[3,4-d]pyrimidine-3-carboxamide,
(S,E)-N-(4-((3-chloro-4-fluorophenyl)amino)-7-((tetrahydrofuran-3-yl)oxy)quinazolin-6-yl)-4-(dimethylamino)but-2-enamide,
N-(2-((2-(dimethylamino)ethyl)(methyl)amino)-4-methoxy-5-((4-(1-methyl-1H-indol-3-yl)pyrimidin-2-yl)amino)phenyl)acrylamide,
N-(3-((2-((4-(4-acetylpiperazin-1-yl)-2-methoxyphenyl)amino)-5-(trifluoromethyl)pyrimidin-4-yl)amino)phenyl)acrylamide,
(E)-N-(4-((3-chloro-4-(pyridin-2-ylmethoxy)phenyl)amino)-3-cyano-7-ethoxyquinolin-6-yl)-4-(dimethylamino)but-2-enamide,
(E)-N-(4-((3-chloro-4-fluorophenyl)amino)-7-methoxyquinazolin-6-yl)-4-(piperidin-1-yl)but-2-enamide,
1-(4-((4-((3,4-dichloro-2-fluorophenyl)amino)-7-methoxyquinazolin-6-yl)oxy)piperidin-1-yl)prop-2-en-1-one,
N-(3-((5-fluoro-2-((4-(2-methoxyethoxy)phenyl)amino)pyrimidin-4-yl)amino)phenyl)acrylamide,
(E)-4-((4-((3-bromo-4-chlorophenyl)amino)pyrido[3,4-d]pyrimidin-6-yl)amino)-N,N-dimethyl-N-((1-methyl-4-nitro-1H-imidazol-5-yl)methyl)-4-oxobut-2-en-1-aminium bromide,
(Z)-3-(3-(3,5-bis(trifluoromethyl)phenyl)-1H-1,2,4-triazol-1-yl)-N'-(pyrazin-2-yl)acrylohydrazide,
(Z)-3-(3-(3,5-bis(trifluoromethyl)phenyl)-1H-1,2,4-triazol-1-yl)-N'-(pyridin-2-yl)acrylohydrazide,
N-((3R,4R)-4-fluoro-1-(6-((3-methoxy-1-methyl-1H-pyrazol-4-yl)amino)-9-methyl-9H-purin-2-yl)pyrrolidin-3-yl)acrylamide,
N-((3S,4S)-3-((6-(2,6-dichloro-3,5-dimethoxyphenyl)quinazolin-2-yl)amino)tetrahydro-2H-pyran-4-yl)acrylamide,
(3E,5E)-1-acryloyl-3,5-bis(4-nitrobenzylidene)piperidin-4-one,
(E)-N-(7-((1R,5S,6s)-3-oxabicyclo[3.1.0]hexan-6-ylethynyl)-4-((3-chloro-4-fluorophenyl)amino)quinazolin-6-yl)-4-(dimethylamino)but-2-enamide,
(3E,5E)-1-acryloyl-3,5-bis(4-fluoro-3-nitrobenzylidene)azepan-4-one,
N-(2-((6-(2,6-dichloro-3,5-dimethoxyphenyl)quinazolin-2-yl)amino)-3-methylphenyl)acrylamide,
(Z)-3-(3-(3,5-bis(trifluoromethyl)phenyl)-1H-1,2,4-triazol-1-yl)-N'-pivaloylacrylohydrazide, and
N-(2-((2-((tetrahydro-2H-pyran-4-yl)amino)-5-(trifluoromethyl)pyrimidin-4-yl)amino)phenyl)acrylamide.

In another embodiment, the drug molecule is a reversible or irreversible covalent kinase inhibitor chosen from:

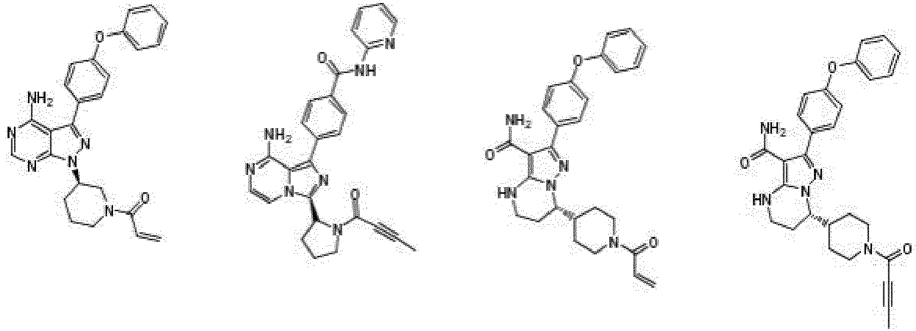

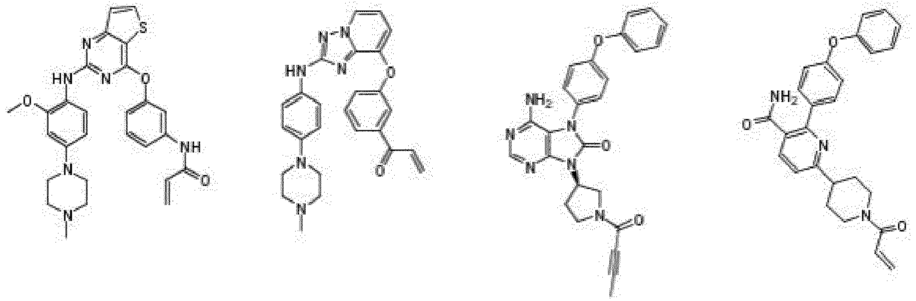

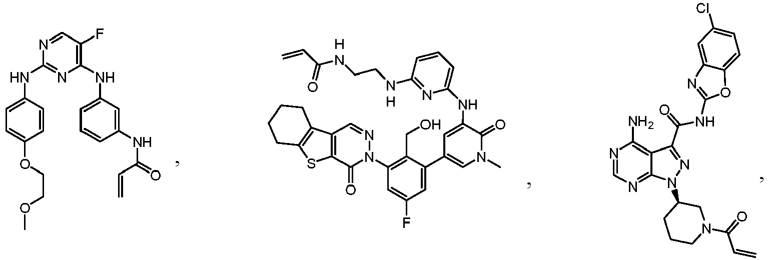

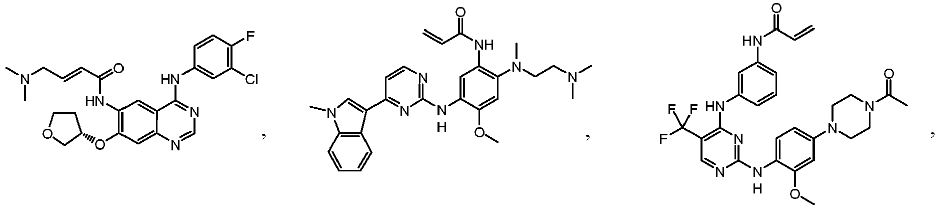

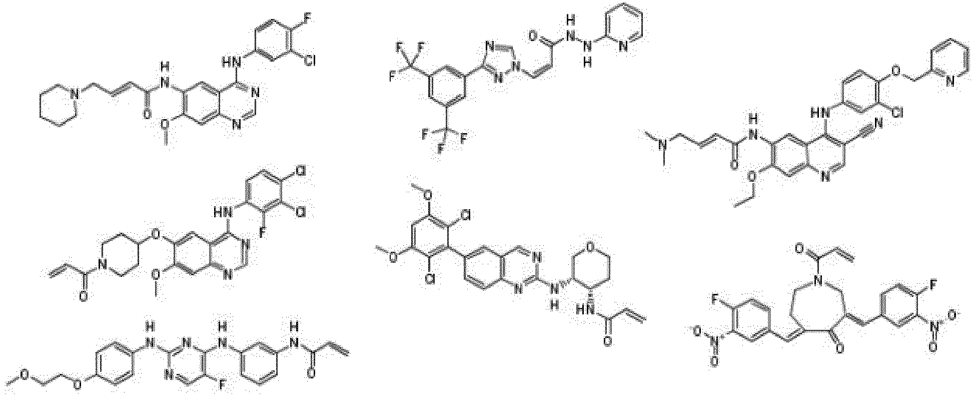

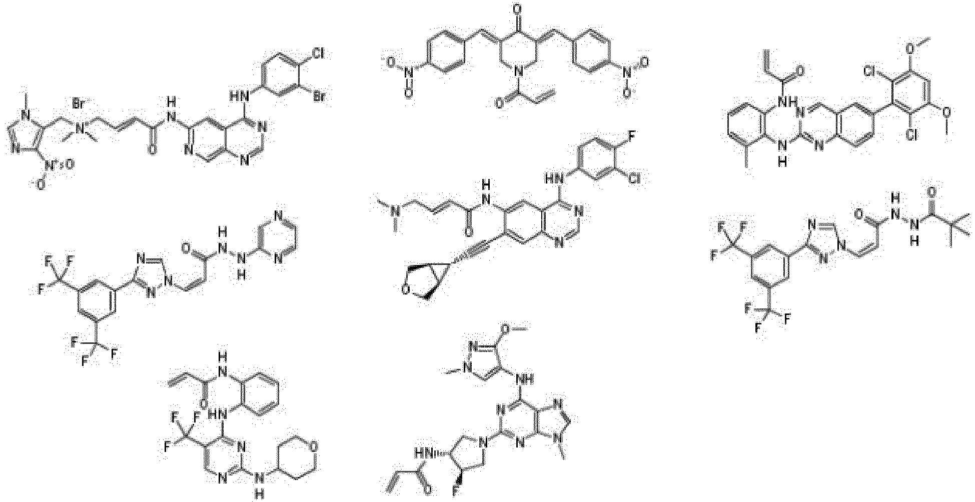

Unless otherwise stated, the following terms used in the specification and claims are defined for the purposes of this Application and have the following meaning:
"Alkyl" as used herein means a linear saturated monovalent hydrocarbon radical of one to six carbon atoms or a branched saturated monovalent hydrocarbon radical of three to six carbon atoms, e.g., methyl, ethyl, propyl, 2-propyl, butyl (including all isomeric forms), pentyl (including all isomeric forms), and the like.

"Substituted alkyl" means alkyl group as defined herein which is substituted with one, two, or three substituents independently selected from hydroxyl, alkoxy, carboxy, cyano, carboxy, alkoxycarbonyl, alkylthio, alkylsulfonyl, halo, -CONRR' or -NRR' (where each R is hydrogen, alkyl, cycloalkyl, hydroxyalkyl, or alkoxyalkyl, and each R' is hydrogen, alkyl, or cycloalkyl) or heterocyclyl (for example heterocycloamino) which is optionally substituted with one or two groups independently selected from alkyl, hydroxyl, alkoxy, alkylthio, alkylsulfonyl, halo, or -CONRR' where R and R' are as defined above.

"Alkynyl" as used herein means a linear saturated monovalent hydrocarbon radical of two to six carbon atoms or a branched saturated monovalent hydrocarbon radical of three to six carbon atoms that contains a triple bond, e.g., ethynyl, propynyl, 2-propynyl, butynyl (including all isomeric forms), pentynyl (including all isomeric forms), and the like.

"Alkylene" as used herein means a linear saturated divalent hydrocarbon radical of one to six carbon atoms or a branched saturated divalent hydrocarbon radical of three to six carbon atoms unless otherwise stated e.g., methylene, ethylene, propylene, 1-methylpropylene, 2-methylpropylene, butylene, pentylene, and the like.

"Alkylthio" as used herein means a -SR radical where R is alkyl as defined above, e.g., methylthio, ethylthio, and the like.

"Alkylsulfonyl" as used herein means a -SO₂R radical where R is alkyl as defined above, e.g., methylsulfonyl, ethylsulfonyl, and the like.

"Amino" means a -NH₂.

"Alkylamino" as used herein means a -NHR radical where R is alkyl as defined above, e.g., methylamino, ethylamino, propylamino, or 2-propylamino, and the like.

"Alkoxy" as used herein means a -OR radical where R is alkyl as defined above, e.g., methoxy, ethoxy, propoxy, or 2-propoxy, *n*-, *iso*-, or *tert*-butoxy, and the like.

"Alkoxyalkyl" as used herein means a linear monovalent hydrocarbon radical of one to six carbon atoms or a branched monovalent hydrocarbon radical of three to six carbons substituted with at least one alkoxy group, for example one or two alkoxy groups, as defined above, e.g., 2-methoxyethyl, 1-, 2-, or 3-methoxypropyl, 2-ethoxyethyl, and the like.

"Alkoxycarbonyl" as used herein means a -C(O)OR radical where R is alkyl as defined above, e.g., methoxycarbonyl, ethoxycarbonyl, and the like.

"Aminocarbonyl" as used herein means a -CONRR' radical where R is independently hydrogen, alkyl, or substituted alkyl, each as defined herein and R' is hydrogen, alkyl, cycloalkyl, cycloalkylalkyl, aryl, aralkyl, heteroaryl, heteroaralkyl, heterocyclyl, heterocyclylalkyl, or substituted alkyl, each as defined herein and wherein the aryl, heteroaryl, or heterocyclyl ring either alone or part of another group e.g., aralkyl, is optionally substituted with one, two, or three substituents independently selected from alkyl, hydroxy, alkoxy, halo, haloalkyl, haloalkoxy, carboxy, alkoxycarbonyl, alkylcarbonyl, cyano, -CONH₂, alkylaminocarbonyl, dialkylaminocarbonyl, or substituted alkylaminocarbonyl, e.g., -CONH₂, methylaminocarbonyl, 2-dimethylaminocarbonyl, and the like. When R is hydrogen and R' is alkyl in -CONRR', the group is also referred to herein as alkylaminocarbonyl and when R and R' are both alkyl in -CONRR', the group is also referred to herein as dialkylaminocarbonyl. When R is hydrogen and R' is substituted alkyl in -CONRR', the group is also referred to herein as substituted alkylaminocarbonyl.

"Aminosulfonyl" as used herein means a -SO₂NRR' radical where R is independently hydrogen, alkyl, or substituted alkyl, each as defined herein and R' is hydrogen, alkyl, cycloalkyl, cycloalkylalkyl, aryl, aralkyl, heteroaryl, heteroaralkyl, heterocyclyl, heterocyclylalkyl, or substituted alkyl, each as defined herein and wherein the aryl, heteroaryl, or heterocyclyl ring either alone or part of another group e.g., aralkyl, is optionally substituted with one, two, or three substituents independently selected alkyl, hydroxy, alkoxy, halo, haloalkyl, haloalkoxy, carboxy, alkoxycarbonyl, alkylcarbonyl, cyano, -CONH₂, alkylaminocarbonyl, dialkylaminocarbonyl, substituted alkylaminocarbonyl, e.g., - SO₂NH₂, methylaminosulfonyl, dimethylaminosulfonyl, and the like. When R is hydrogen and R' is alkyl in -SO₂NRR', the group is also referred to herein as alkylaminosulfonyl and when R and R' are both alkyl in - SO₂NRR', the group is also referred to herein as dialkylaminosulfonyl.

"Acyl" as used herein means a -COR radical where R is alkyl, haloalkyl, cycloalkyl, cycloalkylalkyl, aryl, aralkyl, heteroaryl, heteroaralkyl, heterocyclyl, or heterocyclylalkyl, each as defined herein, and wherein the aryl, heteroaryl, or heterocyclyl ring either alone or part of another group e.g., aralkyl, is optionally substituted with one, two, or three substituents independently selected from alkyl, hydroxy, alkoxy, halo, haloalkyl, haloalkoxy, carboxy, alkoxycarbonyl, alkylcarbonyl, cyano, -CONH₂, alkylaminocarbonyl, dialkylaminocarbonyl, or substituted alkylaminocarbonyl, e.g., acetyl, propionyl, benzoyl, pyridinylcarbonyl, and the like. When R is alkyl, the radical is also referred to herein as alkylcarbonyl.

"Aryl" as used herein means a monovalent monocyclic or bicyclic aromatic hydrocarbon radical of 6 to 10 ring atoms e.g., phenyl or naphthyl.

"Aralkyl" as used herein means a -(alkylene)-R radical where R is aryl as defined above.

"Cycloalkyl" as used herein means a cyclic saturated monovalent hydrocarbon radical of three to ten carbon atoms wherein one or two carbon atoms may be replaced by an oxo group, e.g., cyclopropyl, cyclobutyl, cyclopentyl, or cyclohexyl, and the like.

"Cycloalkylalkyl" as used herein means a -(alkylene)-R radical where R is cycloalkyl as defined above; e.g., cyclopropylmethyl, cyclobutylmethyl, cyclopentylethyl, or cyclohexylmethyl, and the like.

"Cycloalkylene" as used herein means a cyclic saturated divalent hydrocarbon radical of three to ten carbon atoms wherein one or two carbon atoms may be replaced by an oxo group, e.g., cyclopropylene, cyclobutylene, cyclopentylene, or cyclohexylene, and the like.

Cycloalkylene(alkylene)as used herein means a -(alkylene)-R radical where R is cycloalkylene as defined above and one point of attachment of the divalent cycloalkyl radical is to the alkylene moiety.

"Carboxy" means -COOH.

"Disubstituted amino" means a -NRR' radical where R and R' are independently alkyl, cycloalkyl, cycloalkylalkyl, acyl, aryl, aralkyl, heteroaryl, heteroaralkyl, heterocyclyl, heterocyclylalkyl, or substituted alkyl, each as defined herein, and wherein the aryl, heteroaryl, or heterocyclyl ring either alone or part of another group e.g., aralkyl, is optionally substituted with one, two, or three substituents independently selected from alkyl, hydroxy, alkoxy, halo, haloalkyl, haloalkoxy, carboxy, alkoxycarbonyl, alkylcarbonyl, cyano, -CONH₂, alkylaminocarbonyl, dialkylaminocarbonyl, or substituted alkylaminocarbonyl, e.g., dimethylamino, phenylmethylamino, and the like. When the R and R' groups are alkyl, the disubstituted amino group maybe referred to herein as dialkylamino.

"Halo" as used herein means fluoro, chloro, bromo, or iodo, for example fluoro or chloro.

"Haloalkyl" as used herein means alkyl radical as defined above, which is substituted with one or more halogen atoms, for example one to five halogen atoms, for example fluorine or chlorine, including those substituted with different halogens, e.g., -CH₂Cl, -CF₃, -CHF₂, - CH₂CF₃, -CF₂CF₃, -CF(CH₃)₂, and the like. When the alkyl is substituted with only fluoro, it is referred to in this Application as fluoroalkyl.

"Haloalkoxy" as used herein means a -OR radical where R is haloalkyl as defined above e.g., -OCF₃, -OCHF₂, and the like. When R is haloalkyl where the alkyl is substituted with only fluoro, it is referred to in this Application as fluoroalkoxy.

"Hydroxyalkyl" as used herein means a linear monovalent hydrocarbon radical of one to six carbon atoms or a branched monovalent hydrocarbon radical of three to six carbons substituted with one or two hydroxy groups, provided that if two hydroxy groups are present they are not both on the same carbon atom. Representative examples include, but are not limited to, hydroxymethyl, 2-hydroxyethyl, 2-hydroxypropyl, 3-hydroxypropyl, 1-(hydroxymethyl)-2-methylpropyl, 2-hydroxybutyl, 3-hydroxybutyl, 4-hydroxybutyl, 2,3-dihydroxypropyl, 1-(hydroxymethyl)-2-hydroxyethyl, 2,3-dihydroxybutyl, 3,4-dihydroxybutyl and 2-(hydroxymethyl)-3-hydroxypropyl, for example 2-hydroxyethyl, 2,3-dihydroxypropyl, and 1-(hydroxymethyl)-2-hydroxyethyl.

"Heterocyclyl" as used herein means a saturated or unsaturated monovalent monocyclic group of 4 to 8 ring atoms in which one or two ring atoms are heteroatom selected from N, O, or S(O)ₙ, where n is an integer from 0 to 2, the remaining ring atoms being C. The heterocyclyl ring is optionally fused to a (one) aryl or heteroaryl ring as defined herein provided the aryl and heteroaryl rings are monocyclic. The heterocyclyl ring fused to monocyclic aryl or heteroaryl ring is also referred to in this Application as "bicyclic heterocyclyl" ring. Additionally, one or two ring carbon atoms in the heterocyclyl ring can optionally be replaced by a -CO- group. More specifically the term heterocyclyl includes, but is not limited to, pyrrolidino, piperidino, homopiperidino, 2-oxopyrrolidinyl, 2-oxopiperidinyl, morpholino, piperazino, tetrahydropyranyl, thiomorpholino, and the like. When the heterocyclyl ring is unsaturated it can contain one or two ring double bonds provided that the ring is not aromatic. When the heterocyclyl group contains at least one nitrogen atom, it is also referred to herein as heterocycloamino and is a subset of the heterocyclyl group. When the heterocyclyl group is a saturated ring and is not fused to aryl or heteroaryl ring as stated above, it is also referred to herein as saturated monocyclic heterocyclyl.

"Heterocyclylalkyl" as used herein means a -(alkylene)-R radical where R is heterocyclyl ring as defined above e.g., tetraydrofuranylmethyl, piperazinylmethyl, morpholinylethyl, and the like.

"Heterocycloamino" as used herein means a saturated or unsaturated monovalent monocyclic group of 4 to 8 ring atoms in which one or two ring atoms are heteroatom selected from N, O, or S(O)ₙ, where n is an integer from 0 to 2, the remaining ring atoms being C provided that at least one of the ring atoms is N. Additionally, one or two ring carbon atoms in the heterocycloamino ring can optionally be replaced by a -CO- group. When the heterocycloamino ring is unsaturated it can contain one or two ring double bonds provided that the ring is not aromatic.

"Heteroaryl" as used herein means a monovalent monocyclic or bicyclic aromatic radical of 5 to 10 ring atoms where one or more, for example one, two, or three, ring atoms are heteroatom selected from N, O, or S, the remaining ring atoms being carbon. Representative examples include, but are not limited to, pyrrolyl, thienyl, thiazolyl, imidazolyl, furanyl, indolyl, isoindolyl, oxazolyl, isoxazolyl, benzothiazolyl, benzoxazolyl, quinolinyl, isoquinolinyl, pyridinyl, pyrimidinyl, pyrazinyl, pyridazinyl, triazolyl, tetrazolyl, and the like.

"Heteroaralkyl" as used herein means a -(alkylene)-R radical where R is heteroaryl as defined above."Substituted aryl or substituted heteroaryl" means aryl or heteroaryl as defined above, that is substituted with one, two, or three substituents independently selected from hydrogen, alkyl, substituted alkyl, cycloalkyl, cycloalkylalkyl, haloalkyl, haloalkoxy, halogen, amino, monosubstituted amino, disubstituted amino, acyl, aminocarbonyl, aminosulfonyl, -OR', -SR', -OC(O)R', -CO₂R', -NR"C(O)R', -NR"C(O)NR'R", - NR"C(O)₂R', -SO₂R', -NR"SO₂R', -CN, -NO₂, aryl, aralkyl, heteroaryl, heteroaralkyl, heterocyclyl,and heterocyclylalkyl where R' is hydrogen, alkyl, haloalkyl, substituted alkyl, cycloalkyl, cycloalkylalkyl, aryl, aralkyl, heteroaryl, heteroaralkyl, heterocyclyl or heterocyclylalkyl and R" is hydrogen, alkyl, or substituted alkyl; or R' and R" together with the nitrogen atom to which they are attached from heterocycloamino; provided at least one of the three substituent is not hydrogen and furthermore wherein each of the aryl (except in "substituted aryl"), heteroaryl (except in "substituted aryl"), cycloalkyl, heterocycloamino, and heterocyclyl ringin any of the above groups, is optionally substituted with:
(i) one, two, or three substituents independently selected from hydrogen, alkyl, substituted alkyl, cycloalkyl, cycloalkylalkyl, haloalkyl, haloalkoxy, halogen, amino, monosubstituted amino, disubstituted amino, acyl, aminocarbonyl, aminosulfonyl, -OR', -SR', -OC(O)R', -CO₂R', -NR"C(O)R', -NR"C(O)NR'R", -NR"C(O)₂R', -SO₂R', -NR"SO₂R', -CN, -NO₂, aryl, aralkyl, heteroaryl, heteroaralkyl, heterocyclyl, or heterocyclylalkyl where R' is hydrogen, alkyl, haloalkyl, substituted alkyl, cycloalkyl, cycloalkylalkyl, aryl, aralkyl, heteroaryl, heteroaralkyl, heterocyclyl, or heterocyclylalkyl and R" is hydrogen, alkyl, or substituted alkyl; or R' and R" together with the nitrogen atom to which they are attached from heterocycloamino; and furthermore wherein the aryl, heteroaryl, cycloalkyl, heterocycloamino, or heterocyclyl ring in any of the above groups in (i) is substituted with one, two, or three substituents independently selected from hydrogen, alkyl, hydroxy, alkoxy, halo, haloalkyl, haloalkoxy, carboxy, alkoxycarbonyl, alkylcarbonyl, cyano, -CONH₂, alkylaminocarbonyl, dialkylaminocarbonyl, substituted alkylaminocarbonyl, amino, or monosubstituted or disubstituted amino.

"Heteroalkylene" as used herein means an -(alkylene)- radical where one, two or three carbons in the alkylene chain is replaced by -O-, N(H, alkyl, or substituted alkyl), S, SO, SO₂, or CO.

"Monosubstituted amino" as used herein means a -NHR radical where R is alkyl, cycloalkyl, cycloalkylalkyl, acyl, aryl, aralkyl, heteroaryl, heteroaralkyl, heterocyclyl, heterocyclylalkyl, or substituted alkyl, each as defined herein, and wherein the aryl, heteroaryl, or heterocyclyl ring either alone or part of another group e.g., aralkyl, is optionally substituted with one, two, or three substituents independently selected from alkyl, hydroxy, alkoxy, halo, haloalkyl, haloalkoxy, carboxy, alkoxycarbonyl, alkylcarbonyl, cyano, -CONH₂, alkylaminocarbonyl, dialkylaminocarbonyl, or substituted alkylaminocarbonyl,e.g., methylamino, phenylamino, hydroxyethylamino, and the like. When R is alkyl, the monosubstituted amino group maybe referred to herein as alkylamino.

The term "solid oral dosage form" refers to the oral dosage form administered to a patient comprising a enteric coat surrounding a core containing the drug molecule which core optionally comprises excipients, diluents, carriers and coatings.

The term "delayed release" as used herein means the release of the drug molecule from the dosage form until either a predetermined time or site in the GI tract is reached by the dosage form.

The term "cellulose derivative" or "polysaccharide derivative" refers to a cellulose polymer or polysaccharide wherein at least a portion of the hydroxyls on the saccharide repeat units have been reacted to form an ether or ester linkage. Examples include and are not limited to hydroxyalkyl celluloses, hydroxyalkyl alkylcelluloses, and carboxyalkyl cellulose esters, such as hydroxypropyl methylcelluloses (hypromelloses or HPMC), hydroxypropylcelluloses (HPC), and the like.

The term "hydrophilic" for purposes of the present disclosure relates to materials that have affinity towards water.

The term "water soluble" for purposes of the present disclosure relates to materials that dissolve to the extent required, in an aqueous media at a pH of from about 1 to about 8, and is not particularly limited.

The term "water swellable" for purposes of the present disclosure relates to materials that are relatively insoluble in water, but which can absorb water.

Suitable hydrophilic materials comprise water soluble or water swellable materials. Examples of such materials include salts, sugars, and polymers such as hydroxyalkyl celluloses, hydroxyalkyl alkylcelluloses, and carboxyalkyl cellulose esters, for example, hydroxypropyl methylcelluloses (hypromelloses or HPMC), hydroxypropylcelluloses (HPC), and combinations comprising one or more of the foregoing materials. Hydroxypropyl methylcelluloses that are hydrophilic in nature and may be used in the present disclosure are sold in different viscosity grades such as those sold under the brand name Methocel^{™} available from Dow Chemical Co. Examples of hydroxypropyl methylcelluloses of a low viscosity grade include those available under the brand names Methocel E5, Methocel E-15 LV, Methocel E50 LV, Methocel K100 LV and Methocel F50 LV whose 2% by weight aqueous solutions have viscosities of 5 cP, 15 cP, 50 cP, 100 cP, and 50 cP, respectively. Examples of hydroxypropyl methylcelluloses having medium viscosity include those available under the brand names Methocel E4M and Methocel K4M, both of whose 2% by weight aqueous solutions have a viscosity of 4000 cP. Examples of hydroxypropyl methylcellulose polymers having high viscosity include those available under the brand names Methocel K15M and Methocel K100M whose 2% by weight aqueous solutions have viscosities of 15,000 cP and 100,000 cP, respectively. The hydroxypropyl methylcellulose polymers may be present in the pharmaceutical compositions of the present disclosure in amounts from about 0.1 % to 50% by weight.

The hydroxypropylcellulose polymers that may be used in the present disclosure also include, for example, polymers available under the brand name Klucel^{™}, available from Nippon Soda Co. Hydroxypropylcellulose polymers available under the brand names Klucel EF, Klucel LF, Klucel JF and Klucel GF, whose 2% by weight aqueous solutions have viscosities less than 1000 cP, are examples of low viscosity hydrophilic polymers. A hydroxypropylcellulose polymer available under the brand name Klucel ME whose 2% by weight aqueous solution has a viscosity in the range from 4,000-6,500 cP is a medium viscosity hydrophilic polymer. Hydroxypropyl cellulose polymers available sold as HPC-SL, HPC-L, and HPC-M, whose 2% by weight aqueous solutions have viscosities of 3-6 cP, 6-10 cP, and 150-400 cP, respectively, are examples of low viscosity hydrophilic polymers, while HPC-H has a viscosity of 1 ,000-4000 cP and is an example of a medium viscosity hydrophilic polymer. The hydroxypropylcellulose polymers may be present in an amount from about 0.1 % to 50% by weight.

Water swellable materials suitable for making delayed release dosage forms are compounds that are able to expand when they are exposed to aqueous fluids, such as gastro-intestinal fluids. One or more water swellable compounds may be present in a coating and optionally one or more pharmaceutically acceptable excipients.

Suitable compounds which can be used as water swellable substances include, for example, low-substituted hydroxypropyl celluloses, e.g. L-HPC, cross- linked polyvinylpyrrolidones, e.g., PVP-XL, Kollidone^{™} CL and Polyplasdone^{™} XL, sodium carboxymethylcellulose, cross-linked sodium carboxymethylcellulose, e.g., Ac-di-sol^{™} and Primellose^{™}, sodium starch glycolate, e.g., Primojel^{™}, sodium carboxymethylcelluloses, e.g., Nymcel^{™} ZSB10, sodium carboxymethyl starches, e.g., Explotab^{™}, ion-exchange resins, e.g., Dowex^{™} or Amberlite^{™} products, microcrystalline cellulose, e.g., Avicel^{™} products, starches and pregelatinized starches, e.g., Starch 1500^{™} and Sepistab ST200^{™}, formalin- casein, e.g., Plas-Vita^{™}, and combinations comprising one or more of the foregoing water swellable substances.

In some embodiments, hydrophilic materials include polyalkylene oxides, polysaccharide gums, and crosslinked polyacrylic acids. Suitable polyalkylene oxides, such as linear polymers of unsubstituted ethylene oxide, include Polyox^{™} products from The Dow Chemical Company, U.S., having molecular weights about 100,000-7,000,000. Other useful polyalkylene oxide polymers are made from propylene oxide, or mixtures of ethylene oxide and propylene oxide.

Polysaccharide gums, both natural and modified (semi-synthetic), can be used. Examples are dextran, xanthan gum, gellan gum, welan gum and rhamsan gum.

Crosslinked polyacrylic acids that can be used include those having properties similar to those described above for alkyl-substituted cellulose and polyalkylene oxide polymers. Useful crosslinked polyacrylic acids include those with viscosities about 4,000 to about 40,000 cP (for a 1 % aqueous solution at 25°C). Three specific examples are CARBOPOL^{™} grades 971 P, 974P, and 934P (sold by The Lubrizol Corporation, Cleveland, Ohio, USA). Further examples are polymers known as WATER LOCK^{™}, which are starch/acrylate/acrylamide copolymers available from Grain Processing Corporation, Muscatine, Iowa, USA.

The hydrophilicity and water swellability of these polymers cause the subcoat to swell in size after oral administration, due to ingress of water. The release rate of an active agent from the subcoat is primarily dependent upon the rate of water inhibition and the rate at which the active agent dissolves and diffuses from the swollen polymer, which in turn is related to the solubility and dissolution rate of the active agent, the active agent particle size, and/or the active agent concentration in the dosage form.

Suitable "hydrophobic" materials are water-insoluble neutral or synthetic waxes, fatty alcohols such as lauryl, myristyl, stearyl, cetyl, or cetostearyl alcohol, fatty acids and derivatives thereof, including fatty acid esters such as such as glyceryl monostearate, glycerol monooleate, acetylated monoglycerides, stearin, palmitin, laurin, myristin, cetyl esters wax, glyceryl palmitostearate, glyceryl behenate, hydrogenated castor oils, cottonseed oils, fatty acid glycerides (mono-, di-, and tri-glycerides), hydrogenated fats, hydrocarbons, normal waxes, stearic acid, stearyl alcohol, materials having hydrocarbon backbones, and combinations comprising one or more of the foregoing materials. Suitable waxes include, but are not limited to, beeswax, Glycowax^{®} (a N,N'-distearoylethyelenediamine, from Lonza), castor wax, carnauba wax, and wax-like substances.

The term "immediate release dosage form" as used herein refers to a dosage formulation, in liquid or solid form, which releases drug in the stomach and does not have a protective coating to delay contact of the drug with the intestinal mucosa. An aestheric or taste masking coating may be included in the "immediate release dosage form".

The term "core" as used herein refers to all components of the solid oral dosage form that are surrounded by the subcoat (C) and the outer enteric coating (B) which is stable in acidic pH in the stomach and serves to minimize exposure of core containing a drug molecule to the stomach. The core comprises the drug molecule and optionally other excipients, diluents and carriers. The core also may optionally comprise additional coating(s) other than the subcoat (C) and the outer enteric coating (B).

The term "enteric coating" as used herein refers to a pH sensitive polymeric coating which prevents or minimizes release or dissolution of drug molecule in the stomach but allows release in the small intestine. Enteric coatings are used to protect the drug molecule from complete or partial degradation in the acidic environment of the stomach.

The term "simulated intestinal fluid" as used herein refers to the dissolution media described in United States Pharmacopeia 33-28NF (2010) and European Pharmacopeia 7.0 (2010).

The terms, "coat", "subcoat", "coating", "film", "layer", "covering", "membrane" and the like are interchangeable.

The term "channel" as used herein refers to a pathway in a coating or subcoating permitting the uptake of water into the core and/or efflux of the drug molecule through the coat or subcoat. In some embodiments the uptake in water can result in swelling of the core allowing influx of water or efflux of the drug molecule. In embodiments in which the subcoat is a water insoluble polymer which allows influx of water resulting in sufficient sewlling of the core to rupture the subcoat covering the core thereby releasing the drug molecule inhibitor as a bolus.

Cₘₐₓ and AUC are parameters used to assess bioavailability of a drug molecule. The term Cₘₐₓ as used herein means the maximum plasma concentration of the drug molecule achieved after a single dose administration of the dosage form. The phrase "area under the curve" (AUC) as used herein refers to the area under a curve (*i*.*e*., the integral) of a plot of concentration of drug concentration in blood plasma against time. The AUC (from zero to infinity) represents the total drug exposure over time and is proportional to the total amount of drug absorbed by the body (*i*.*e*., the total amount of drug that reaches the blood circulation) assuming linear pharmacokinetics.

The phrase "pharmaceutically acceptable" indicates that the substance or composition is compatible chemically and/or toxicologically, with the other ingredients comprising a formulation, and/or the mammal being treated therewith.

Mammal as used herein means domesticated animals such as dogs, cats, horses and humans. Preferably a human patient.

The term "pharmaceutically acceptable salt" of a compound means a salt that is pharmaceutically acceptable and that possesses the desired pharmacological activity of the parent compound. Such salts include: (1) acid addition salts, formed with inorganic acids such as hydrochloric acid, hydrobromic acid, sulfuric acid, nitric acid, phosphoric acid, and the like; or formed with organic acids such as acetic acid, propionic acid, hexanoic acid, cyclopentanepropionic acid, glycolic acid, pyruvic acid, lactic acid, malonic acid, succinic acid, malic acid, maleic acid, fumaric acid, tartaric acid, citric acid, benzoic acid, 3-(4-hydroxybenzoyl)benzoic acid, cinnamic acid, mandelic acid, methanesulfonic acid, ethanesulfonic acid, 1,2-ethane-disulfonic acid, 2-hydroxyethanesulfonic acid, benzenesulfonic acid, 4-chlorobenzenesulfonic acid, 2-naphthalenesulfonic acid, 4-toluenesulfonic acid, camphorsulfonic acid, 4-methylbicyclo[2.2.2]-oct-2-ene-1-carboxylic acid, glucoheptonic acid, 3-phenylpropionic acid, trimethylacetic acid, tertiary butylacetic acid, lauryl sulfuric acid, gluconic acid, glutamic acid, hydroxynaphthoic acid, salicylic acid, stearic acid, muconic acid, and the like; or (2) salts formed when an acidic proton present in the parent compound either is replaced by a metal ion, e.g., an alkali metal ion, an alkaline earth ion, or an aluminum ion; or coordinates with an organic base such as ethanolamine, diethanolamine, triethanolamine, tromethamine, N-methylglucamine, and the like.

The term "enteric coating" as used herein refers to a pH sensitive polymeric coating which prevents or minimizes release or dissolution of drug molecule in the stomach but allows release in the small intestine and include but not limited to, acrylate, methacrylate and ethacrylate polymers and copolymers, cellulose derivatives and polyvinyl acetates. The properties of acrylate, methacrylate and ethacrylate polymers (primarily their solubility in biological fluids) can vary based on the degree and type of substitution. Examples of suitable acrylic polymers include methacrylic acid copolymers and ammonium methacrylate copolymers. The Eudragit series L, S, and RS ( manufactured Rohm Pharma and known as Evonik^{®}) are available as solubilized in organic solvent, aqueous dispersion, or dry powders. The Eudragit series RL, NE, and RS are insoluble in the gastrointestinal tract but are permeable and are used primarily for colonic targeting. The Eudragit series L, L-30D and S are insoluble in stomach and dissolve in the intestine. The term poly(meth)acrylate means polyacrylate polymers , polymethacrylate polymers or copolymers containing both acrylic and methacrylic acid.

Examples of suitable cellulose derivatives include ethyl cellulose and reaction mixtures of partial acetate esters of cellulose with phthalic anhydride. The performance can vary based on the degree and type of substitution. Cellulose acetate phthalate (CAP) dissolves in pH > 6. Aquateric (FMC) is an aqueous based system and is a spray dried CAP pseudolatex with particles < 1 µm. Other components in Aquateric can include Pluronics^{®}, Tweens^{®}, and acetylated monoglycerides. Other suitable cellulose derivatives include; cellulose acetate trimellitate (CAT, Eastman); cellulose acetate succinate (CAS), methylcellulose (Pharmacoat, Methocel^{™}); hydroxypropylmethyl cellulose phthalate (HPMCP); hydroxypropylmethyl cellulose succinate (HPMCS); and hydroxypropylmethylcellulose acetate succinate (HPMCAS e.g., AQOAT (Shin Etsu)). The performance can vary based on the degree and type of substitution. For example, HPMCP such as, HP-50, HP-55, HP-55S, HP-55F grades are suitable. Suitable grades of hydroxypropylmethylcellulose acetate succinate include, but are not limited to, AS-LG (LF), which dissolves at pH 5, AS-MG (MF), which dissolves at pH 5.5, and AS-HG (HF), which dissolves at higher pH. These polymers are offered as granules, or as fine powders for aqueous dispersions.

Poly Vinyl Acetate Phthalate (PVAP) dissolves in pH greater than 5, and it is much less permeable to water vapor and gastric fluids. Detailed description of above polymers and their pH-dependent solubility can be found at in the article titled "Enteric coated hard gelatin capsules" by Professor Karl Thoma and Karoline Bechtold at http://pop.www.capsugel.com/media/library/enteric-coated-hard-gelatin-capsules.pdf.

Shellac, also called purified lac, it is a refined product obtained from the resinous secretion of an insect. This coating dissolves in media of pH>7. Zein is class of prolamine protein found in maize. Zein is clear, odorless, tasteless, hard, water-insoluble, and edible, and is used as a coating in pharmaceutical formulations.

In one embodiment, the enteric coating is made from acrylic acid, methacrylic acid or ethacrylic acid polymers or copolymers, cellulose acetate (and its succinate and phthalate derivatives), hydroxypropyl methyl cellulose phthalate, polyvinyl acetate phthalate, hydroxyethyl ethyl cellulose phthalate, cellulose acetate tetrahydrophtalate, acrylic resin or shellac. In another embodiment the polymer is chosen from cellulose acetate phthalate (CAP; dissolves above pH 6), polyvinyl acetate phthalate (PVAP, disintegrates at pH 5), hydroxypropyl methyl cellulose phthalate (HPMCP, grade HP50 disintegrates at pH 5 and HP50 disintegrates at 5.5), methylacrylic acid copolymers (Eudragit L 100 and L12.5 disintegrate between about 6 and about 7, Eudragit L-30 and L100-55 disintegrate at pH greater than 5.5 and Eudragit S100, S12.5 and FS 30D disintegrate at pH greater than 7).

The enteric coating can, and usually does, contain a plasticizer. Suitable plasticizers include triethyl citrate (Citroflex 2), triacetin (glyceryl triacetate), acetyl triethyl citrate (Citroflec A2), Carbowax 400 (polyethylene glycol 400), diethyl phthalate, tributyl citrate, acetylated monoglycerides, glycerol, fatty acid esters, propylene glycol, and dibutyl phthalate. In particular, anionic carboxylic acrylic polymers usually will contain 10-25% by weight of a plasticizer, especially dibutyl phthalate, polyethylene glycol (PEG), triethyl citrate and triacetin. The amount of plasticizer is optimized for each enteric coating layer formula, in relation to the selected enteric coating layer polymer(s), selected plasticizer(s) and the applied amount of said polymer(s), in such a way that the mechanical properties, i.e. flexibility and hardness of the enteric coating layer(s), for instance exemplified as Vickers hardness, are adjusted so that if a tablet is desired the acid resistance of the pellets covered with enteric coating layer(s) does not decrease significantly during compression of pellets into tablets. The amount of plasticizer is usually above 5% by weight of the enteric coating layer polymer(s), (In one embodiment the amount of plasticizer is 15-50%. In another embodiment the amount of plasticizer is 20-50%). The maximum thickness of the applied enteric coating is normally only limited by processing conditions and the desired dissolution profile.

Formulations disclosed herein contain, unless stated otherwise, one or more pharmaceutically acceptable excipient(s) such as binders, surfactants, diluents, buffering agents, anti adherents, glidants, hydrophilic or hydrophobic polymers, retardants, stabilizing agents or stabilizers, disintegrants or superdisintegrants, dispersants, antioxidants, antifoaming agents, fillers, flavors, colorants, lubricants, sorbents, preservatives, plasticizers, or sweeteners, or mixtures thereof, which facilitate processing of the drug molecule (or embodiments thereof disclosed herein) or a pharmaceutically acceptable salt thereof into preparations which can be used pharmaceutically. The pharmaceutically acceptable excipients can be in the coating and/or the core. Any of the well-known techniques and excipients may be used as suitable and as understood in the art, *see* for example, Remington: The Science and Practice of Pharmacy, Twenty-first Ed., (Pharmaceutical Press, 2005); Liberman, H. A., Lachman, L., and Schwartz, J.B. Eds., Pharmaceutical Dosage Forms, Vol. 1-2 Taylor & Francis 1990; and R.I. Mahato, Ansel's Pharmaceutical Dosage Forms and Drug Delivery Systems, Second Ed. (Taylor & Francis, 2012).

Additives such as dispersants, colorants, pigments polymers (*e*.*g*. poly(ethylacrylate, methylmethacrylate), anti-tacking and anti-foaming agents may also be included into the enteric coating layer(s). Other compounds may be added to increase film thickness and to decrease diffusion of acidic gastric juices into the acid susceptible material.

In some embodiments, the formulations may include one or more pH adjusting agents or buffering agents, for example, acids such as acetic, boric, citric, lactic, phosphoric and hydrochloric acids; bases such as sodium hydroxide, sodium phosphate, sodium borate, sodium citrate, sodium acetate, sodium lactate and *tris*-hydroxymethylaminomethane; and buffers such as citrate/dextrose, sodium bicarbonate, ammonium chloride, and the like. Such acids, bases and buffers are included in an amount required to maintain pH of the composition in an acceptable range.

In some embodiments, the formulations may also include one or more salts in an amount required to bring osmolality of the composition into an acceptable range. Such salts include those having sodium, potassium or ammonium cations and chloride, citrate, ascorbate, borate, phosphate, bicarbonate, sulfate, thiosulfate or bisulfite anions; suitable salts include sodium chloride, potassium chloride, sodium thiosulfate, sodium bisulfite and ammonium sulfate.

In some embodiments, the formulations may also include one or more antifoaming agents to reduce foaming during processing which can result in coagulation of aqueous dispersions, bubbles in the finished film, or generally impair processing. Exemplary anti-foaming agents include silicon emulsions or sorbitan sesquoleate.

In some embodiments, the formulations may also include one or more antioxidants, such as non-thiol antioxidants, for example, butylated hydroxytoluene (BHT), sodium ascorbate, ascorbic acid, and tocopherol. In certain embodiments, antioxidants enhance chemical stability where required.

In some embodiments, the formulations may also include one or more preservatives to inhibit microbial activity. Suitable preservatives include mercury-containing substances such as merfen and thiomersal; stabilized chlorine dioxide; and quaternary ammonium compounds such as benzalkonium chloride, cetyltrimethylammonium bromide and cetylpyridinium chloride.

In some embodiments, the formulations may also include one or more binders. Binders impart cohesive qualities and include, *e.g*., alginic acid and salts thereof; cellulose derivatives such as carboxymethylcellulose, methylcellulose (*e*.*g*., Methocel^{®}), hydroxypropylmethylcellulose, hydroxyethylcellulose, hydroxypropylcellulose (*e.g*., Klucel^{®}), ethylcellulose (*e*.*g*., Ethocel^{®}), and microcrystalline cellulose (*e*.*g*., Avicel^{®}); microcrystalline dextrose; amylose; magnesium aluminum silicate; polysaccharide acids; bentonites; gelatin; polyvinyl-pyrrolidone/vinyl acetate copolymer; crosspovidone; povidone; starch; pregelatinized starch; tragacanth, dextrin, a sugar, such as sucrose (*e*.*g*., Dipac^{®}), glucose, dextrose, molasses, mannitol, sorbitol, xylitol (*e*.*g*., Xylitab^{®}), and lactose; a natural or synthetic gum such as acacia, tragacanth, ghatti gum mucilage of isapol husks, polyvinylpyrrolidone (*e*.*g*., Polyvidone^{®} CL, Kollidon^{®} CL, Polyplasdone^{®} XL-10), larch arabogalactan, Veegum^{®}, polyethylene glycol, polyethylene oxide, waxes, sodium alginate, and the like.

In general, binder levels of about 10 to about 70% are used in powder-filled gelatin capsule formulations. Binder usage level in tablet formulations varies whether direct compression, wet granulation, roller compaction, or usage of other excipients such as fillers which itself can act as moderate binder. Formulators skilled in art can determine the binder level for the formulations, but binder usage level of up to 70% in tablet formulations is common.

In some embodiments, the formulations may also include dispersing agents and/or viscosity modulating agents. Dispersing agents and/or viscosity modulating agents include materials that control the diffusion and homogeneity of a drug through liquid media or a granulation method or blend method. In some embodiments, these agents also facilitate the effectiveness of a coating or eroding matrix. Exemplary diffusion facilitators/dispersing agents include, *e.g*., hydrophilic polymers, electrolytes, Tween^{®} 20, 60 or 80, PEG, polyvinylpyrrolidone (PVP; commercially known as Plasdone^{®}), and the carbohydrate-based dispersing agents such as, for example, hydroxypropyl celluloses (*e*.*g*., HPC, HPC-SL, and HPC-L), hydroxypropyl methylcelluloses (*e.g*., HPMC K100, RPMC K4M, HPMC K15M, and HPMC K100M), carboxymethylcellulose sodium, methylcellulose, triethylcellulose, hydroxyethyl-cellulose, hydroxypropyl-cellulose, hydroxypropylmethylcellulose phthalate, hydroxypropyl-methylcellulose acetate stearate (HPMCAS), noncrystalline cellulose, magnesium aluminum silicate, triethanolamine, polyvinyl alcohol (PVA), vinyl pyrrolidone/vinyl acetate copolymer (S630), 4-(1,1,3,3-tetramethylbutyl)-phenol polymer with ethylene oxide and formaldehyde (also known as tyloxapol), poloxamers (*e.g.*, Pluronics^{®} F68, F88, and F108, which are block copolymers of ethylene oxide and propylene oxide); and poloxamines (e.g., Tetronic^{®} 908, also known as Poloxamine^{®} 908, which is a tetrafunctional block copolymer derived from sequential addition of propylene oxide and ethylene oxide to ethylenediamine (BASF Corporation, Parsippany, N.J.)), polyvinylpyrrolidone K12, polyvinylpyrrolidone K17, polyvinylpyrrolidone K25, or polyvinylpyrrolidone K30, polyvinylpyrrolidone/vinyl acetate copolymer (S-630), polyethylene glycol, e.g., the polyethylene glycol can have a molecular weight of about 300 to about 6000, or about 3350 to about 4000, or about 7000 to 5400, sodium carboxymethylcellulose, methylcellulose, polysorbate-80, sodium alginate, gums, such as, *e*.*g*., gum tragacanth and gum acacia, guar gum, xanthans, including xanthan gum, sugars, cellulosics, such as, e.g., sodium carboxymethylcellulose, methylcellulose, sodium carboxymethylcellulose, polysorbate-80, sodium alginate, polyethoxylated sorbitan monolaurate, polyethoxylated sorbitan monolaurate, povidone, carbomers, polyvinyl alcohol (PVA), alginates, chitosans and combinations thereof.

In some embodiments, the formulations may also include one or more "diluents" which refers to chemical compounds that are used to dilute the compound of interest prior to delivery. Diluents can also be used to stabilize compounds because they can provide a more stable environment Salts dissolved in buffered solutions (which also can provide pH control or maintenance) are utilized as diluents in the art, including, but not limited to a phosphate buffered saline solution. In certain embodiments, diluents increase bulk of the composition to facilitate compression or create sufficient bulk for homogenous blend for capsule filling. Such compounds include e.g., lactose, starch, mannitol, sorbitol, dextrose, microcrystalline cellulose such as Avicel^{®}; dibasic calcium phosphate, dicalcium phosphate dihydrate; tricalcium phosphate, calcium phosphate; anhydrous lactose, spray-dried lactose; pregelatinized starch, compressible sugar, such as Di-Pac^{®} (Amstar); hydroxypropyl-methylcellulose, hydroxypropylmethylcellulose acetate stearate, sucrose-based diluents, confectioner's sugar; monobasic calcium sulfate monohydrate, calcium sulfate dihydrate; calcium lactate trihydrate, dextrates; hydrolyzed cereal solids, amylose; powdered cellulose, calcium carbonate; glycine, kaolin; mannitol, sodium chloride; inositol, bentonite, and the like.

In some embodiments, the formulation may contain surface active agents or surfactants are long chain molecules that can accumulate at hydrophilic/hydrophobic (water/oil) interfaces and lower the surface tension at the interface. As a result they can stabilise an emulsion. In some embodiments, the surfactant may comprise: Tween^{®} (polyoxyethylene sorbate) family of surfactants, Span^{®} (sorbitan long chain carboxylic acid esters) family of surfactants, Pluronic^{®} (ethylene or propylene oxide block copolymers) family of surfactants, Labrasol^{®}, Labrafil^{®} and Labrafac^{®}(each polyglycolyzed glycerides) families of surfactants, sorbitan esters of oleate, stearate, laurate or other long chain carboxylic acids, poloxamers (polyethylene-polypropylene glycol block copolymers or Pluronic^{®}), other sorbitan or sucrose long chain carboxylic acid esters, mono and diglycerides, PEG derivatives of caprylic/capric triglycerides and mixtures thereof or mixture of two or more of the above. In some embodiments the surfactant phase may comprise a mixture of Polyoxyethylene (20) sorbitan monooleate (Tween 80^{®}) and sorbitan monooleate (Span 80^{®}).

In some embodiments, the formulations may also include one or more "disintegrant" which includes both the dissolution and dispersion of the dosage form when contacted with gastrointestinal fluid. "Disintegration agents or disintegrants" facilitate the breakup or disintegration of a substance. Examples of disintegration agents include a starch, e.g., a natural starch such as corn starch or potato starch, a pregelatinized starch such as National 1551 or sodium starch glycolate such as Promogel^{®} or Explotab^{®}, a cellulose such as a wood product, methyl crystalline cellulose, *e.g*., Avicel^{®}, Avicel^{®} PH101, Avicel^{®} PH 102, Avicel^{®} PH105, Elceme^{®} P100, Emcocel^{®}, Vivacel^{®}, and Solka-Floc^{®}, methylcellulose, croscarmellose, or a cross-linked cellulose, such as cross-linked sodium carboxymethylcellulose (Ac-Di-Sol^{®}), cross-linked carboxymethylcellulose, or cross-linked croscarmellose, a cross-linked starch such as sodium starch glycolate, a cross-linked polymer such as crosspovidone, a cross-linked polyvinylpyrrolidone, alginate such as alginic acid or a salt of alginic acid such as sodium alginate, a clay such as Veegum^{®} HV (magnesium aluminum silicate), a gum such as agar, guar, locust bean, Karaya, pectin, or tragacanth, sodium starch glycolate, bentonite, a natural sponge, a surfactant, a resin such as a cation-exchange resin, citrus pulp, sodium lauryl sulfate, sodium lauryl sulfate in combination starch, and the like.

In some embodiments, the formulations may also include erosion facilitators. "Erosion facilitators" include materials that control the erosion of a particular material in gastrointestinal fluid. Erosion facilitators are generally known to those of ordinary skill in the art. Exemplary erosion facilitators include, *e.g*., hydrophilic polymers, electrolytes, proteins, peptides, and amino acids.

In some embodiments, the formulations may also include one or more filling agents which include compounds such as lactose, calcium carbonate, calcium phosphate, dibasic calcium phosphate, calcium sulfate, microcrystalline cellulose, cellulose powder, dextrose, dextrates, dextran, starches, pregelatinized starch, sucrose, xylitol, lactitol, mannitol, sorbitol, sodium chloride, polyethylene glycol, and the like.

In some embodiments, the formulations may also include one or more flavoring agents and/or "sweeteners" *e.g*., acacia syrup, acesulfame K, alitame, anise, apple, aspartame, banana, Bavarian cream berry, black currant, butterscotch, calcium citrate, camphor, caramel, cherry, cherry cream chocolate, cinnamon, bubble gum, citrus, citrus punch, citrus cream, cotton candy, cocoa, cola, cool cherry, cool citrus, cyclamate, cylamate, dextrose, eucalyptus, eugenol, fructose, fruit punch, ginger, glycyrrhetinate, glycyrrhiza (licorice) syrup, grape, grapefruit, honey, isomalt, lemon, lime, lemon cream, monoammonium glyrrhizinate, maltol, mannitol, maple, marshmallow, menthol, mint cream, mixed berry, neohesperidine DC, neotame, orange, pear, peach, peppermint, peppermint cream, powder, raspberry, root beer, rum, saccharin, safrole, sorbitol, spearmint, spearmint cream, strawberry, strawberry cream, stevia, sucralose, sucrose, sodium saccharin, saccharin, aspartame, acesulfame potassium, mannitol, talin, sylitol, sucralose, sorbitol, Swiss cream, tagatose, tangerine, thaumatin, tutti fruitti, vanilla, walnut, watermelon, wild cherry, wintergreen, xylitol, or any combination of these flavoring ingredients, *e.g*., anise-menthol, cherry-anise, cinnamon-orange, cherry-cinnamon, chocolate-mint, honey-lemon, lemon-lime, lemon-mint, menthol-eucalyptus, orange-cream, vanilla-mint, and mixtures thereof.

In some embodiments, the formulations may also include one or more lubricants and glidants which are compounds that prevent, reduce or inhibit adhesion or friction of materials. Exemplary lubricants include, *e.g*., stearic acid, calcium hydroxide, talc, sodium stearyl fumerate, a hydrocarbon such as mineral oil, or hydrogenated vegetable oil such as hydrogenated soybean oil, higher fatty acids and their alkali-metal and alkaline earth metal salts, such as aluminum, calcium, magnesium, zinc, stearic acid, sodium stearates, glycerol, talc, waxes, boric acid, sodium benzoate, sodium acetate, sodium chloride, leucine, a polyethylene glycol (*e.g*., PEG4000) or a methoxypolyethylene glycol such as Carbowax^{®}, sodium oleate, sodium benzoate, glyceryl behenate, polyethylene glycol, magnesium or sodium lauryl sulfate, colloidal silica such as Syloid^{®}, Cab-O-Sil^{®}, a starch such as corn starch, silicone oil, a surfactant, and the like.

In some embodiments, the formulations may also include one or more solubilizers which include compounds such as triacetin, triethylcitrate, ethyl oleate, ethyl caprylate, sodium lauryl sulfate, sodium doccusate, vitamin E TPGS, dimethylacetamide, N-methylpyrrolidone, N-hydroxyethylpyrrolidone, polyvinylpyrrolidone, hydroxypropylmethyl cellulose, hydroxypropyl cyclodextrins for example Captisol^{®}, ethanol, n-butanol, isopropyl alcohol, cholesterol, bile salts, polyethylene glycol 200-600, glycofurol, transcutol, propylene glycol, and dimethyl isosorbide and the like. In one embodiment, the solubilizer is vitamin E TPGS and/or Captisol^{®}.

In some embodiments, the formulations may also include one or more suspending agents which include compounds such as polyvinylpyrrolidone, *e.g*., polyvinylpyrrolidone K112, polyvinylpyrrolidone K17, polyvinylpyrrolidone K25, or polyvinylpyrrolidone K30, vinyl pyrrolidone/vinyl acetate copolymer (S630), polyethylene glycol, *e.g*., the polyethylene glycol can have a molecular weight of about 300 to about 6000, or about 3350 to about 4000, or about 7000 to about 5400, sodium carboxymethylcellulose, methylcellulose, hydroxypropylmethylcellulose, hydroxymethylcellulose acetate stearate, polysorbate-80, hydroxyethylcellulose, sodium alginate, gums, such as, *e.g*., gum tragacanth and gum acacia, guar gum, xanthans, including xanthan gun, sugars, cellulosics, such as, *e.g*., sodium carboxymethylcellulose, methylcellulose, sodium carboxymethylcellulose, hydroxypropylmethylcellulose, hydroxyethylcellulose, polysorbate-80, sodium alginate, polyethoxylated sorbitan monolaurate, polyethoxylated sorbitan monolaurate, povidone and the like.

In some embodiments, the formulations may also include one or more wetting agents which include compounds such as oleic acid, glyceryl monostearate, sorbitan monooleate, sorbitan monolaurate, triethanolamine oleate, polyoxyethylene sorbitan monooleate, polyoxyethylene sorbitan monolaurate, sodium docusate, sodium oleate, sodium lauryl sulfate, sodium doccusate, triacetin, Tween 80, vitamin E TPGS, ammonium salts and the like.

In an embodiment, the solid oral dosage form is able to release less than 10% by weight of the compound and/or said pharmaceutically acceptable salt thereof of the core (A) in 1.5 hours in a dissolution vessel comprising an aqueous solution at a pH of less than 3 or a pH of from 4.5 to 5.5, and the solid oral dosage form is able to release not less than 80% by weight of said compound and/or said pharmaceutically acceptable salt thereof of the core (A), from twenty minutes to two hours in a dissolution vessel comprising an aqueous solution at a pH of from 6.4 to 7.4.

In one aspect of this embodiment, the core (A) is able to release not less than 80% by weight of the compound and/or the pharmaceutically acceptable salt thereof of the core (A) from twenty minutes to two hours in a dissolution vessel comprising an aqueous solution at a pH of from 6.4 to 7.

In another aspect of this embodiment, the solid oral dosage form is able to release less than 10% by weight of the compound and/or said pharmaceutically acceptable salt thereof of the core (A) in 1.5 hours in a dissolution vessel comprising an aqueous solution at a pH of from 5.1 to 5.5. The solid oral dosage form may release less than 20% in 1.5 hours in a dissolution vessel comprising an aqueous solution at a pH of from 5.1 to 5.5. The solid oral dosage form may release less than 25% in 1.5 hours in a dissolution vessel comprising an aqueous solution at a pH of from 5.1 to 5.5. The solid oral dosage form may release less than 10% in 1.5 hours in a dissolution vessel comprising an aqueous solution at a pH below 5.1.The solid oral dosage form may release less than 25% by weight of said compound and/or said pharmaceutically acceptable salt thereof in 15 minutes in a dissolution vessel comprising an aqueous solution at a pH of from 6.4 to 7.4. The solid oral dosage from may release less than 25% of aid compound and/or said pharmaceutically acceptable salt thereof in 15 minutes in a dissolution vessel comprising an aqueous solution at a pH of from 6.4 to 7.4. The solid oral dosage form may release less than 35% of said compound and/or said pharmaceutically acceptable salt thereof in 15 minutes in a dissolution vessel comprising an aqueous solution at a pH from 6.4 to 7.4.

In another aspect of this embodiment, the solid oral dosage form is able to release less than 80% by weight of the compound and/or said pharmaceutically acceptable salt thereof of the core (A) in 30 minutes in a dissolution vessel comprising an aqueous solution at a pH of from 6.4 to 7.4. The solid oral dosage form may release less than 80% of said compound and/or said pharmaceutically acceptable salt thereof in 30 minutes in a dissolution vessel comprising an aqueous solution at a pH of from 6.4 to 7.4. The solid oral dosage form may release less than 85% of said drug molecule in 30 minutes in a dissolution vessel comprising an aqueous solution at a pH of from 6.4 to 7.4.

In another aspect of this embodiment, the solid oral dosage form is able to release less than 80% by weight of the compound and/or said pharmaceutically acceptable salt thereof of the core (A) in 45 minutes in a dissolution vessel comprising an aqueous solution at a pH of from 6.4 to 7.4. The solid oral dosage form may release less than 80% of said compound and/or said pharmaceutically acceptable salt thereof in 45 minutes in a dissolution vessel comprising an aqueous solution at a pH of from 6.4 to 7.4.

In another aspect of this embodiment, the solid oral dosage form is able to release less than 80% of the compound and/or said pharmaceutically acceptable salt thereof of the core (A) in 60 minutes in a dissolution vessel comprising an aqueous solution at a pH of from 6.4 to 7.4. The solid oral dosage form may release less than 80% of said compound and/or said pharmaceutically acceptable salt thereof in 60 minutes in a dissolution vessel comprising an aqueous solution at a pH of from 6.4 to 7.4.

In another aspect of this embodiment, the solid oral dosage form is able to release at least 80% of the compound and/or said pharmaceutically acceptable salt thereof of the core (A) in 120 minutes in a dissolution vessel comprising an aqueous solution at a pH of from 6.4 to 7.4. The solid oral dosage form may release at least 80% of said compound and/or said pharmaceutically acceptable salt thereof in 60 minutes in a dissolution vessel comprising an aqueous solution at a pH of from 6.4 to 7.4.

In another aspect of this embodiment, in each of the tests described in the forgoing aspects, the aqueous solution is a simulated intestinal fluid at a pH of from 6.4 to 7.4.

In another aspect of this embodiment, the solid oral dosage form comprises a pharmaceutically acceptable acid salt of the compound of the core (A). Alternatively, the solid oral dosage form may comprise the free base of the compound of the core (A).

In another aspect of this embodiment, the core (A) of the solid oral dosage form comprises a pharmaceutically acceptable salt of the compound and sufficient additional pharmaceutically acceptable acid to enhance dissolution of said compound and/or said pharmaceutically acceptable salt thereof.

In another aspect of this embodiment, the core (A) of the solid oral dosage form comprises a pharmaceutically acceptable salt of the compound and a pharmaceutically acceptable acid in sufficient quantity to produce an acidic aqueous solution within the solid oral dosage form prior to the release of the compound and/or the pharmaceutically acceptable salt thereof.

In another aspect of this embodiment, the core (A) of the solid oral dosage form comprises a pharmaceutically acceptable salt of the compound and a surfactant present in a concentration above its critical micelle concentration upon disintegration in 50 ml of aqueous media. The surfactant may be present in a concentration above its critical micelle concentration upon disintegration in 20 mL of aqueous media.

In another aspect of this embodiment, the compound and/or the pharmaceutically acceptable salt thereof of the core (A) is a solid wherein the mean particle size is from 0.3 micron to 100 microns.

In another aspect of this embodiment, the compound and/or the pharmaceutically acceptable salt thereof of the core (A) is a solid wherein the mean particle size is from 1 micron to 50 microns.

In another aspect of this embodiment, the compound and/or the pharmaceutically acceptable salt thereof of the core (A) is a solid wherein the mean particle size is less than or equal to 15 micron.

In another aspect of this embodiment, the enteric coating (B) is from 10% to 150% of the weight of the core.

In another aspect of this embodiment, the enteric coating (B) is from 20% to 100% of the weight of the core.

In another aspect of this embodiment, the enteric coating (B) is from 30% to 60% of the weight of the core.

In another aspect of this embodiment, the enteric coating (B) is from 5 to 500 microns thick.

In another aspect of this embodiment, the enteric coating (B) is from 8 to 150 microns thick.

In another aspect of this embodiment, the enteric coating (B) is from 50 to 100 microns thick.

In another aspect of this embodiment, the enteric coating (B) is selected from polymerized gelatin, shellac, methacrylic acid copolymer type CNF, cellulose butyrate phthalate, cellulose hydrogen phthalate, cellulose proprionate phthalate, polyvinyl acetate phthalate (PVAP), cellulose acetate phthalate (CAP), cellulose acetate trimellitate (CAT), hydroxypropyl methylcellulose phthalate, hydroxypropyl methylcellulose acetate, dioxypropyl methylcellulose succinate, carboxymethyl ethylcellulose (CMEC), hydroxypropyl methylcellulose acetate succinate (HPMCAS), and (meth)acrylic acid polymers and copolymers, wherein said polymers are made from one monomer and said copolymers are made from two or more monomers, wherein said monomers are selected from any of methyl acrylate, ethyl acrylate, methyl methacrylate, and ethyl methacrylate.

In another aspect of this embodiment, the enteric coating comprises a poly(meth)acrylate polymer.

In another aspect of this embodiment, the enteric coating comprises a Eudragit^{®} L or S series polymer.

In another aspect of this embodiment, the enteric coating comprises a Eudragit^{®} L100, L12.5, S100, S12.5, orFS 30D.

In another aspect of this embodiment, the enteric coating comprises a cellulose derivative.

In another aspect of this embodiment, the cellulose derivative is selected from methylcellulose, cellulose acetate phthalate, hydroxymethyl cellulose phthalate (HPMCP), hydroxypropyl methyl cellulose succinate (HPMCS), and hydroxymethyl cellulose acetate succinate (HPMCAS).

In another aspect of this embodiment, the enteric coating comprises a polyvinyl acetate phthalate (PVAP) polymer.

In another aspect of this embodiment, the subcoat (C) is a water soluble or hydrophilic erodible polymer, said polymer being a low molecular weight polymer selected from hydroxymethyl cellulose (HPMC), hydroxyethyl cellulose, hydroxymethyl cellulose, hydroxypropyl cellulose, microcrystalline cellulose, polyvinylpyrrolidiones, polysaccharides (or a polysaccharide derivative), polyvinyl alcohols, polyethylene glycol (PEG), a polypropylene glycol (PPG), and a PEG-PPG block copolymer.

In another aspect of this embodiment, the subcoat (C) is a water insoluble composition comprising (i) particles of a water soluble compound capable of forming channels in the water insoluble composition, or (ii) water insoluble hydrophilic particles which cause swelling of the subcoat when in contact with an aqueous media.

In another aspect of this embodiment, the subcoat (C) comprises particles of a water soluble compound capable of forming channels.

The term "water insoluble hydrophilic particles" as used herein include but is not limited to, polysaccharides including particles of calcium pectinate, calcium alginate, calcium xanthate, any metal salt of a polysaccharide containing an acid group where the salt renders the polysaccharide insoluble in water, microcrystalline starch, insoluble starch, any water insoluble polysaccharide (*e.g*., cellulose or microcrystalline cellulose), any covalently crosslinked polysaccharide where said crosslinking renders the polysaccharide insoluble in water. Such crosslinking agents include, but are not limited to, glutaraldehyde, formaldehyde, epichlorohydrin, diacid chlorides, diisocyananates, diacid anhydrides and diamines.

In another aspect of this embodiment, the subcoat (C) comprises water insoluble hydrophilic particles which cause swelling of the subcoat when in contact with an aqueous media.

In another aspect of this embodiment, the subcoat (C) is a water insoluble composition and comprises particles of a water soluble compound forming channels allowing influx of water into the solid oral dosage form and diffusion of the compound and/ or said pharmaceutically acceptable salt thereof into the intestine.

In another aspect of this embodiment, the subcoat (C) is a water insoluble composition such as a polymer comprising particles of a water soluble compound capable of forming channels that are impermeable to said compound and/or said pharmaceutically acceptable salt thereof, but allows entry of water and swelling and rupturing of the subcoat and causing release of the compound and/or the pharmaceutically acceptable salt thereof.

The solid oral dosage form is a tablet or in a capsule.

The solid oral dosage form may comprise a pharmaceutically acceptable excipient selected from binders, surfactants, diluents, buffers, anti adherents, glidants, disintegrants, antioxidants, antifoaming agents, fillers, flavors, colors, lubricants, sorbents, preservatives, plasticizers, and sweeteners.

In an embodiment, the AUC resulting from administration of the solid oral dosage is at least about 500% greater than the AUC resulting from administration of an immediate release dosage form having an equivalent amount of the compound and/or said pharmaceutically acceptable salt thereof.

In an embodiment, the AUC resulting from administration of the solid oral dosage is at least about 100% greater than the AUC resulting from administration of an immediate release dosage form having an equivalent amount of the compound and/or said pharmaceutically acceptable salt thereof.

In an embodiment, the AUC resulting from administration of the solid oral dosage is at least about 50% greater than the AUC resulting from administration of an immediate release dosage form having an equivalent amount of the compound and/or said pharmaceutically acceptable salt thereof.

In an embodiment, the solid oral dosage form of the invention is for use in a method of treating a disease treatable by inhibition of BTK, in a patient in recognized need thereof, wherein said method comprises administering to said patient in single or multiple doses, a therapeutically effective amount of the compound and/or the pharmaceutically acceptable salt thereof of the core (A).

In one aspect of this embodiment, the disease is selected from an autoimmune disease, cancer, and an inflammatory disease.

In another aspect of this embodiment, the disease is a leukemia or lymphoma.

In another aspect of this embodiment, the disease is selected from chronic lymphocytic leukemia (CLL), small lymphocytic leukemia (SLL), multiple myeloma, mantle cell lymphoma , and B-cell non-Hodgkin lymphoma.

In an embodiment, the solid oral dosage form of the invention is for use in a method of treating a disease treatable by inhibition of BTK, in a patient in recognized need thereof, wherein said method comprises administering to said patient a drug molecule of the core (A) and having an onset of release of said BTK in jejunum or ileum of the patient, wherein the average systemic bioavailability of the drug molecule as measured by plasma AUC resulting from administration of said pharmaceutical dosage form is about 10%, 20%, 30%, 40%, 50%, 60% or 70% more than the average systemic bioavailability of an immediate release formulation having an equivalent amount of said drug molecule or a pharmaceutically salt thereof salt thereof.

The term "immediate release formulation" as used herein refers to a formulation which is bioavailable as soon as it is administered and has nor protective coatings to delay contact with the intestinal mucosa.

In an embodiment, the solid oral dosage form of the invention is for use in a method of treating a disease treatable by inhibition of BTK, in a patient in recognized need thereof, wherein said method comprises administering to said patient a drug molecule of the core (A) and having an onset of release of said BTK in jejunum and/or ileum of the patient, wherein the average systemic bioavailability of the drug molecule as measured by plasma AUC resulting from administration of said pharmaceutical dosage form is about 10%, 20%, 30%, 40%, 50%, 60% or 70% more than the average systemic bioavailability of an immediate release formulation having an equivalent amount of said drug molecule or a pharmaceutically salt thereof salt thereof administered to the patient after a 4 hour fast.

In one embodiment, seeds or beads (*e.g*., SODAS^{®}) layered with the active agent, optionally mixed with alkaline substances or buffer, can be used as the core material for the further processing. The seeds which are to be layered with the active agent can be water insoluble seeds comprising different oxides, celluloses, organic polymers and other materials, alone or in mixtures or water-soluble seeds comprising different inorganic salts, sugars, non-pareils and other materials, alone or in mixtures. Further, the seeds may comprise the active agent in the form of crystals, agglomerates, compacts etc. The size of the seeds is not essential for the present disclosure but may vary between approximately 0.1 and 4 mm, such as less than 2 mm. The seeds layered with the active agent are produced either by powder or solution/suspension layering using for instance granulation or spray coating layering equipment. The seeds may be covered by a enteric coating or another subcoat.

Before the seeds are layered, the active ingredient may be mixed with further components. Such components can be binders, surfactants fillers, disintegrating agents, alkaline additives or other and/or pharmaceutically acceptable ingredients alone or in mixtures.

In another embodiment, the drug molecule can be optionally mixed with suitable diluents, carriers and excipients to obtain preferred handling and processing properties and a suitable concentration of the active agent and the core then produced by extrusion/spheronization, balling or compression utilizing conventional process equipment. The size of the formulated core material is approximately between 0.1 and 4 mm, such as between 0.1 and 2 mm. The manufactured core material can further be layered with additional ingredients comprising the active agent and/or be used for further processing. Alternatively, the aforementioned core material can be prepared by using spray drying or spray congealing technique.

The core may optionally be covered with one or more separating layer(s) comprising pharmaceutical excipients before applying the enteric coating layer(s) onto the core material in the form of individual pellets. This/these separating layer(s) are formulated to afford specific desired properties to the solid formulation and contain, for example, sugar, PEGs, polyvinylpyrrolidone, polyvinyl alcohol, polyvinyl acetate, hydroxypropyl cellulose, methylcellulose, ethylcellulose, hydroxypropyl methyl cellulose, carboxymethylcellulose sodium, water soluble salts of enteric coating polymers and others, used alone or in mixtures. Additives such as plasticizers, colorants, pigments, fillers anti-tacking and anti-static agents, such as for instance magnesium stearate, titanium dioxide, talc and other additives may also be included into the separating layer(s).

When the optional separating layer, is applied to the core material it may constitute a variable thickness. The maximum thickness of the separating layer(s) is normally only limited by processing conditions. The separating layer may serve as a diffusion barrier and may act as a pH-buffering zone. The optionally applied separating layer(s) is not essential. However, the separating layer(s) may improve the chemical stability of the active substance and/or the physical properties of the novel multiple unit tableted dosage form.

Alternatively, the separating layer may be formed *in situ* by a reaction between an enteric coating polymer layer applied on the core material and an alkaline reacting compound in the core material. Thus, the separating layer formed comprises a water soluble salt formed between the enteric coating layer polymer(s) and an alkaline reacting compound which is in the position to form a salt.

The coating and or separating layers are applied onto the core material or onto the core material covered with separating layer(s) by using any suitable coating technique. The coating layer(s) may optionally further be covered with one or more over-coating layer(s). The over-coating layer(s) can be applied to the enteric coating layered pellets by coating or layering procedures in suitable equipment such as coating pan, coating granulator or in a fluidized bed apparatus using water and/or organic solvents for the coating or layering process. As an alternative the separating layer(s) can be applied to the core material by using powder coating technique. The over-coating layer may further prevent potential agglomeration of enteric coating layered pellets, further it may protect the enteric coating layer towards cracking during the compaction process and enhance the tableting process. The maximum thickness of the applied over-coating layer(s) is normally limited by processing conditions and the desired dissolution profile. The over-coating layer may also be used as a tablet film coating layer.

Pharmaceutical preparations disclosed herein also include capsules made of gelatin, as well as soft, sealed capsules made of gelatin and a plasticizer, such as glycerol or sorbitol. Capsules may also be made of polymers such as hypromellose. The capsules can contain the active ingredients in the coated core as described above. The capsule may additionally contain lactose, binders such as starches, and/or lubricants such as talc or magnesium stearate and, optionally, stabilizers. In soft capsules, the active compounds may be dissolved or suspended in suitable liquids, such as fatty oils, liquid paraffin, lipids, solubilizers, or liquid polyethylene glycols. In addition, stabilizers may be added. All formulations for oral administration should be in dosages suitable for such administration.

It can be desirable to incorporate the drug in the water phase of an emulsion. Such "water-in-oil" emulsion provide a suitable biophysical environment for the drug and can provide an oil-water interface that can protect the drug from adverse effects of pH or enzymes that can degrade the drug. Additionally, such water-in-oil formulations can provide a lipid layer, which can interact favorably with lipids in cells of the body, and can increase the partition of the formulation into the membranes of cells. Such partition can increase the absorption of drugs in such formulations into the circulation and therefore can increase the bioavailability of the drug. The aqueous phase may optionally comprise the active agent suspended in water and a buffer.

In some embodiments, the water-in-oil emulsion contains an oily phase composed of C₈₋₂₂ saturated carboxylic acids or unsaturated carboxylic acids or esters with up to three unsaturated bonds (also branching) or esters or alcohols thereof, a surfactant or a surface active agent, and an aqueous phase containing primarily water and the active agent.

In some embodiments, the solid dosage forms described herein are non-enteric time-delayed release dosage forms. The term "non-enteric time-delayed release" as used herein refers to the delivery so that the release of the drug can be accomplished at some generally predictable location in the intestinal tract more distal to that which would have been accomplished if there had been no delayed release alterations. In some embodiments, the method for delay of release is a coating that becomes permeable, dissolves, ruptures, and/or is no longer intact after a designed duration.

The coating in the time-delayed release dosage forms can have a fixed time to erode after which the drug is released (suitable coatings include polymeric coating such as HPMC, and the like) or has a core comprised of a disinegrant(s) or osmotic agent(s) such as a salt, hydrophilic polymer, typically polyethylene oxide or an alkylcellulose, sugar, or the like, which draw(s) water through a membrane or a gas generating agent such as citric acid and sodium bicarbonate. The membrane may rupture after the swelling pressure exceeds a certain threshold over a desired delay time. Alternatively, a membrane could become porous by leaching an aqueous extractable over a desired delay time. The time delayed dosage forms are sometimes administered in a fasted state to avoid variability in gastric emptying in the fed state.

In one aspect of the invention, the solid oral dosage form contains a core with a water insoluble or relatively water-insoluble rigid coating around a drug-containing swellable core. The coating consists of a hydrophobic polymer that resists water entry into the tablet. The coating is embedded with water soluble or water insoluble hydrophilic particles that are capable of swelling or forming channels through which aqueous solution enters the tablet. The design is such that the coating determines the rate of water uptake while the swelling of the core, which depends on the rate of water uptake and on the swelling properties of the core itself, determines the time of breach of the coating.

The properties of the core further give it the characteristic that it disintegrates after breach of the coating, giving a burst of drug release at a predetermined site in a gastrointestinal tract. The drug may be embedded in the core material or otherwise associated with the core material, for example by dry admixture, or wet granulation. The enteric coated core can be in the form of a matrix tablet or a capsule containing the drug. The core can be in the form of pellets of the pure drug. Alternatively, the core can contain pellets of the drug layered onto a separate core material. Alternatively, the core can contain microcapsules that contain the drug material. More than one of these forms can be present and more than one drug can be delivered in the same delivery system. In all of these forms, release of drug from the core is effective. The core has the essential characteristics of being capable of absorbing sufficient liquid so that it swells considerably, and disintegrates rapidly after the coating is breached. By "swelling considerably" is intended that sufficient swelling occurs so as to bring about and result in a pressure that initiates and/or otherwise facilitates disintegration. By "disintegrating rapidly" is intended that the disintegration occurs essentially in a burst, the burst being sufficient to release efficacious amounts of the drug from the solid oral dosage form.

In various embodiments, the methods and compositions directed to ileo-jejunal delivery of reversible covalent drug molecules are provided in the form of timed release formulations and can be coupled with an immediate release component in a unitary dosage form. The immediate release component can be formulated by any known method such as a layer that envelops the timed release component or the like.

In some embodiments, the compositions described herein improve the tolerability of reversible or irreversible covalent kinase inhibitors. For example, in one non-limiting embodiment, the compositions described herein reduce or eliminate potential adverse side effects of reversible or irreversible covalent kinase inhibitors such as diarrhea and emesis, commonly called vomiting.

The following examples illustrate the preparation of compositions within the scope of the invention. These compositions are provided to enable those skilled in the art to more clearly understand and to practice the present invention. They should not be considered as limiting the scope of the invention, but merely as being illustrative and representative thereof.

### Example 1:

### Osmotically Activated Dosage Form for Ileo-jejunal Delivery

The following ingredients are used to prepare an ileo-jejunal dosage form for the BTK inhibitor, (R)-2-(3-(4-amino-3-(2-fluoro-4-phenoxyphenyl)-1H-pyrazolo[3,4-d]pyrimidin-1-yl)piperidine-1-carbonyl)-4-methyl-4-(4-(oxetan-3-yl)piperazin-1-yl)pent-2-enenitrile:

| Core layer 1 | |
|---|---|
| | mg |
| Active Ingredient | 100 |
| citric acid | 30 |
| lactose | 50 |
| Cabosil^{®} (DSM) | 3.5 |
| Sodium stearoyl fumarate | 3.5 |

| Core layer 2 (osmotic core) | |
|---|---|
| | mg |
| PolyOx^{™} Coagulant (Dow) | 400 |
| NaCl | 100 |
| Sodium stearoyl fumarate | 3.5 |

| semipermeable membrane | |
|---|---|
| | mg |
| Cellulose acetate NF-398-10 | 2.5 |
| Cellulose acetate NF-320S | 17 |
| Hypomellose USP | 1 |
| Polyethylene glycol 3350 NF | 1 |

| enteric coating | |
|---|---|
| | mg |
| Acyleeze^{®} coating solution (Colorcon) | 20% |
| Double distilled water | 100 |

The blend for each core layer is sized and dry blended with the lubricant added last with additional mixing. The 2 blends are tableted by conventional tableting techniques on a rotary bilayer tablet press. A pre-coat of hypromellose solution may optionally also be applied to the uncoated cores. The blends for the tablets are layered in the tablet press and compressed into a tablet with a single compression.

The ingredients for the semipermeable membrane are added to a methylene chloride:methanol (4:1 w/w) solution using a propeller mixer. The uncoated tablets are spray coated and dried.

These coated tablets are then enteric coated with the aqueous solution for different ranges until tablets resist release in acid media for 2 hours, typically with weight gains from 6 to 15% and coating thicknesses ranging from about 50 to about 150 microns.

### Example 2:

### Enteric Coated Tablet for Ileo-jejunal Delivery

200 mg active ingredient for each coated tablet is first sized and then dry blended with 7 mg sodium croscarmellose (Ac-Disol^{®}) and 143 mg of microcrystalline cellulose (Avicel^{®} PH 101) in a V-blender for 20 to 30 minutes. Following this 5 mg of sodium stearyl fumarate is added as a lubricant and blended for 4 to 5 minutes. The blend is that tableted on a rotary tablet press using 5/16" standard concave punches. A coating mixture is prepared from 250 g Eudragit^{®} L-30 D-55, 7.5 g triethyl citrate, 37.5 g talc, and 205 g deionized water. The tablet cores are placed in a perforated pan coater rotated at 15 rpm at 40°C. This mixture is sprayed with an inlet air temperature of 44 to 48°C , an exhaust air temperature of 29 to 32°C, a product temperature of 26°C, rotating at 30 to 32 rpm, spray pressure of 20 psi, and an airflow of 30-32 CFM. After curing for 30 minutes with an air inlet temperature of 60°C and rotating at 15 rpm, the heat is turned off and the tablets cooled to room temperature while rotating. The amount of weight gain after coating is about 5 to about 15%, and typically about 10%. Dissolution times for the enteric coating at pH 6.8 were targeted at greater than 80% in 1 hour.

### Example 3:

### Enteric Coated Granules

Enteric coated granules of a size range from 300 to 500 microns are incorporated for inclusion in capsules, either in gelatin or hypromellose capsules, in sachets or in stickpacks, or in an oral suspension. The active ingredient and low viscosity hypromellose (about 2%) are sized and mixed in a V blender, and then added to a fluid bed granulator. Granules are formed by spraying dilute aqueous polyvinylpyrrolidone solution on to the powder, and then drying the granules in the fluids bed at 45 °C. In the fluid bed, these dried granules are then coated with an Opadry^{®} clear solution in water to provide a seal coat and dried. Eudragit^{®} L-30 D-55 as an aqueous dispersion of 30% polymer, 0.7% sodium lauryl sulfate, and 2.3% Tween^{®} 20 are combined with the plasticizers, triethyl citrate and glyceryl monostearate, and coated on the powder in the fluid bed. After drying the final composition of the enteric-coated granules is about 81.8% active ingredient, about 1.5% hypromellose, about 0.5% Opadry Clear, about 14.5% methacrylic acid copolymer, 1.45% triethyl citrate, and 0.25% glyceryl monostearate. The dried granules are filled into size 0 hypromellose capsules. Dissolution of the capsules at pH 2 shows less than 2% release at 2 hours and the capsule releases greater than 80% of the theoretical drug load in about 45 minutes.

### Example 4:

### Ileo-jejunal Dosing of (R)-2-(3-(4-amino-3-(2-fluoro-4-phenoxyphenyl)-1H-pyrazolo[3,4-d]pyrimidin-1 -yl)piperidine-1 -carbonyl)-4-methyl-4-(4-(oxetan-3 -yl)piperazin-1 -yl)pent-2-enenitrile in Rats

(R)-2-(3-(4-amino-3-(2-fluoro-4-phenoxyphenyl)-1H-pyrazolo[3,4-d]pyrimidin-1-yl)piperidine-1-carbonyl)-4-methyl-4-(4-(oxetan-3-yl)piperazin-1-yl)pent-2-enenitrile was dosed in 10 mg/mL aqueous citrate solution at 20 mg/kg to Wister-Hans rats. Anhydrous citric acid (0.5 equivalents) was added to the free base of (R)-2-(3-(4-amino-3-(2-fluoro-4-phenoxyphenyl)-1H-pyrazolo[3,4-d]pyrimidin-1-yl)piperidine-1-carbonyl)-4-methyl-4-(4-(oxetan-3-yl)piperazin-1-yl)pent-2-enenitrile. The experiment was repeated 3 times each with 2 separate arms, and one oral gavage arm was included in each study. Intra-jejunal (IJ) and intra-duodenal dosing was done in cannulated rats. In the first study, there was a 40-fold increase in the AUC for the U-dosed group compared to the PO group, and the metabolite present in the U-group was reduced 10-fold. The 3 studies are summarized in Figure 1. It is shown that ID dosing increased the AUC and Cₘₐₓ of (R)-2-(3-(4-amino-3-(2-fluoro-4-phenoxyphenyl)-1H-pyrazolo[3,4-d]pyrimidin-1-yl)piperidine-1-carbonyl)-4-methyl-4-(4-(oxetan-3-yl)piperazin-1-yl)pent-2-enenitrile compared to PO dosing, and that IJ dosing substantially increased the AUC and Cₘₐₓ compared to PO dosing in both studies.

### Example 5:

### In Vitro Permeation in Ussing Chambers through Different Intestinal Regions of Rat

Reagents: 10X Krebs-Ringer buffer (KRB) comprised of 1.26M NaCl (Promega, Madison, WI), 25mM KCl, 250mM NaHCO₃, 12mM NaH₂PO₄, 12mM MgCl₂, 25mM CaCl₂ and 18g/L D-Glucose (all from Sigma Aldrich, St. Louis, MO) prepared and stored individually to prevent crystallization. Dilute to 1X working concentration prior to use and pH adjusted with either carbogen perfusion for 20mins to pH 7.4 (oxygenated KRB) or 1M HCl (Alfar Aesar, Ward Hill, MA) to pH 3.5, 6.5 or 7.4. 0.1% Phenol red, Antipyrine and Atenolol were purchased from Sigma Aldrich, St. Louis, MO.

Harvest and preparation of intestinal tissue: 4 to 9 month old adult female virgin Sprague-Drawley rats (Harlan Laboratories, Livermore,CA) were acclimated in-house for a minimum of 3 days prior to experimentation. Adolescent and geriatric animals should be avoided to prevent age-related differences in gastro-intestinal morphology and function. Animals were housed under IACUC-approved husbandry protocols including 12-hour light and 12-hour dark cycles, and allowed standard chow and water *ad libitum.* Tissue harvest should be performed one animal at a time to maintain tissue viability. One at a time, rats were anaesthetized in a sealed gas chamber perfused slowly with 10% (v/v)/min carbon dioxide to minimize distress on the nasal mucosa for approximately 3-5 minutes or until complete sedation as evidenced by lack of response to toe-pinch and shallow breathing. Rats are then quickly euthanized by cervical dislocation.

A modified literature protocol was utilized (D. I. Kosik-Bogacka, et al., (2011) "The effect of L-ascorbic acid and/or tocopherol supplementation on electrophysiological parameters of the colon of rats chronically exposed to lead" Med. Sci .Monit. 2011 17(1):BR16-26). A midline incision was performed to expose the abdominal cavity. The length of the gastro-intestinal tissue from the stomach through the ascending colon was dissected in one piece and placed in a pre-cooled dissection pan filled with ice-cold oxygenated KRB at pH 7.4. Tissues were quickly moved into a fresh dissection pan filled with ice-cold KRB at pH7.4 and continuously bubbled with carbogen gas (95% O₂, 5% CO₂)(CryoSpec, South San Francisco, CA). The different segments of intestine were then separated with a scalpel according to the schematics The stomach comprised *ca.*1.5 inches, the duodenum *ca.* 2 inches, the jejunum *ca.* 8-10 inches, ileum ca. 1 inch and the ascending colon, *ca.* 1 inch.

Each tubular segment was then cut longitudinally along the mesenteric border to expose the luminal surface. Intestinal contents were gently flushed away with care to not disturb the luminal epithelia. Each segment was further cut into smaller pieces measuring approximately 8mm x 10mm, gently stretched and mounted on the pins on one half of a vertical diffusion Ussing chamber (Navicyte^{®}, Harvard Apparatus Inc., Holliston, MA) with an effective surface area of 0.49cm². Mounted segments were visually inspected for tears before fusing with the second half of the chamber. The chambers were immediately filled with 5ml physiological pH matched KRB (Mucosal chambers: Stomach pH 3.5, Duodenum pH 6.5, Jejunum pH 6.5, Ileum pH 7.4, Colon pH 6.5; Serosal chambers: All sections at pH 7.4). Each completed Ussing chamber assembly was serially mounted onto a thermocirculated heat block maintained at 37°C and attached to a gas manifold continuously sparged with carbogen gas at a rate of 3-5 bubbles/second. Phenol red (10 uL 0.1%) was added to each mucosal chamber to check for pH equilibration and evidence of microscopic tears in tissue.

Experimental Protocol: All compounds (experimental samples and control samples) were initially prepared as a 10 mM stock in 100% DMSO. Experimental outline was performed according to (S. Haslam, et al., "Intestinal Ciprofloxacin Efflux: The Role of Breast Cancer Resistance Protein (ABCG2)" Drug Met. Disp. 2011 39(12):2321-28). Briefly, an identical volume of KRB was then removed and replaced by the volume of each compound to obtain a 100 uM starting concentration. Antipyrine and atenolol were included in every experiment as internal reference controls with a maximum of 5 total compounds (including reference controls) in each experiment. Samples (100 uL) were removed from the mucosal chamber at t=0 and t=150min, whilst 100ul samples were removed from the serosal chamber at t=30, 60, 90 and 150 min. The samples were placed in a 96-well plate, frozen at -80°C for analysis by RapidFire-LC-MS/MS. Apparent permeability, Pₐₚₚ was expressed as Pₐₚₚ= (dQ/dt).(1/(A.C₀)) where dQ/dt is the rate of transport from mucosal to serosal chamber, A is the effective surface area of tissue and C₀ is the initial mucosal concentration (A. Sjoberg, et al., "Comprehensive study on regional human intestinal permeability and prediction of fraction absorbed of drugs using the Ussing chamber technique" Eur. J Pharm. Sci. 2013 48: 166-180).

The results for a series of reversible covalent inhibitors are shown in Figure 2. There is generally increased permeability in the distal regions of the GI tract compared to either the stomach or the duodenum, and in particular, the statistically increased permeabilities for these BTK inhibitors and some analogs in the jejunal, ileal, and colonic regions.

### Example 6:

### Dissolution Testing

Dissolution Testing is carried out in accordance with section 711 of United States Pharmacopeia (http://www.pharmacopeia.cn/v29240/usp29nf24s0_c711h.html) using apparatus 2 (paddle at 75 rpm) with sinker alternative 2A. Baths containing 900 mL for both acid (pH 2 or 3) and pH 6.8 tests at temperature 37 °C (± 0.5 °C).

The features disclosed in the foregoing description, or the following claims, expressed in their specific forms or in terms of a means for performing the disclosed function, or a method or process for attaining the disclosed result, as appropriate, may, separately, or in any combination of such features, be utilized for realizing the invention in diverse forms thereof.

The foregoing invention has been described in some detail by way of illustration and example, for purposes of clarity and understanding. It will be obvious to one of skill in the art that changes and modifications may be practiced within the scope of the appended claims. Therefore, it is to be understood that the above description is intended to be illustrative and not restrictive.

Any conflict between any reference cited herein and the specific teachings of this specifications shall be resolved in favor of the latter. Likewise, any conflict between an art-understood definition of a word or phrase and a definition of the word or phrase as specifically taught in this specification shall be resolved in favor of the latter.

## Claims

1. A solid oral dosage form, which is a tablet or capsule, comprising:
(A) a core comprising a compound chosen from: 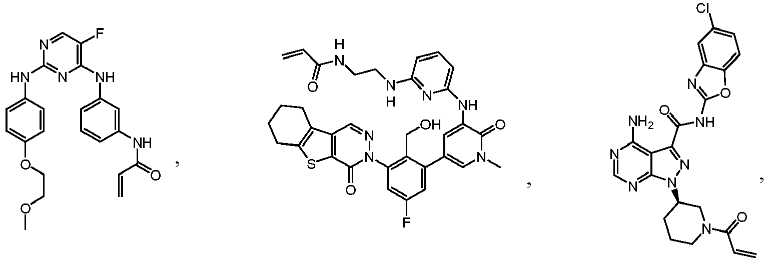 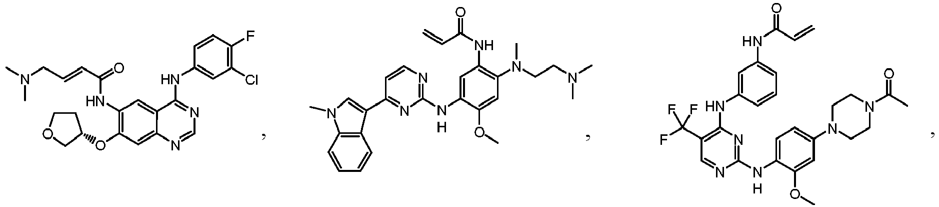 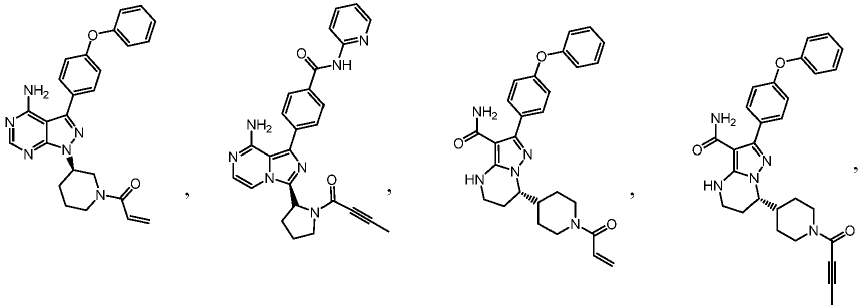 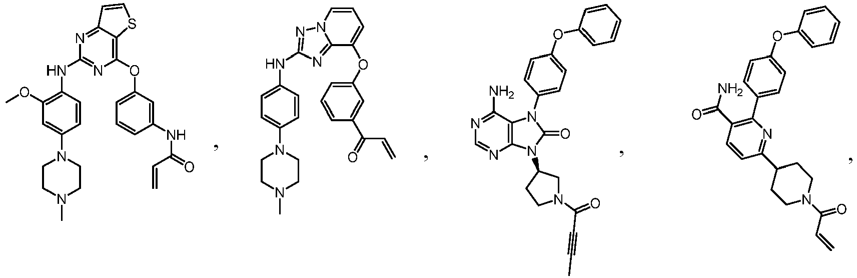 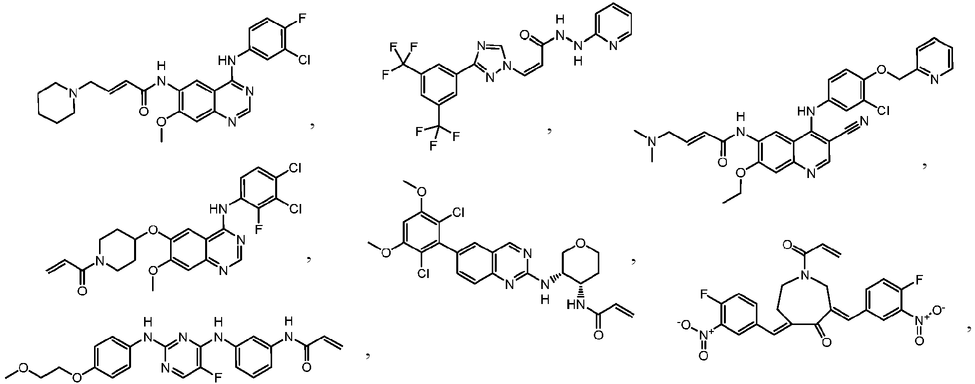 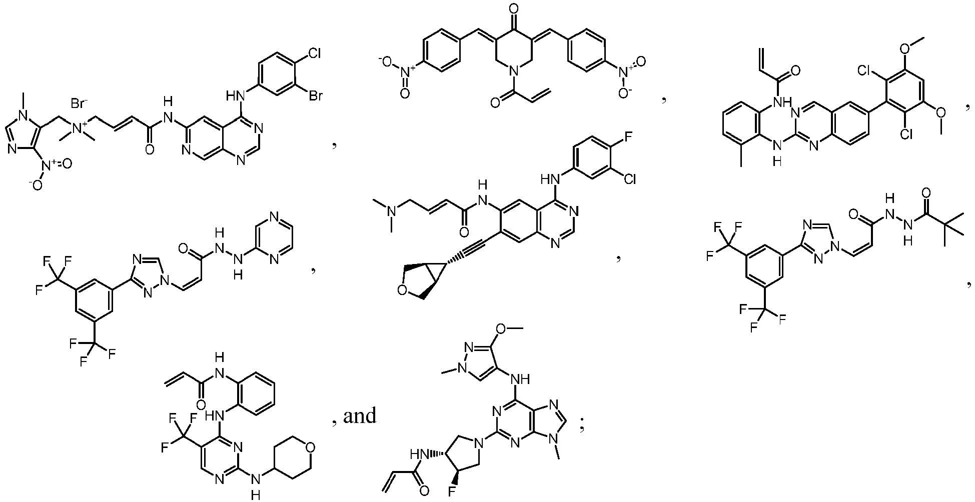 and/or a pharmaceutically acceptable salt thereof;
(B) an enteric coating;
(C) a subcoat between the enteric coating and the core, wherein the subcoat is:
(1) a water soluble or hydrophilic erodible polymer, said polymer being a low molecular weight polymer selected from hydroxymethyl cellulose (HPMC), hydroxyethyl cellulose, hydroxymethyl cellulose, hydroxypropyl cellulose, microcrystalline cellulose, polyvinylpyrrolidiones, polysaccharides (or a polysaccharide derivative), polyvinyl alcohols, polyethylene glycol (PEG), polypropylene glycol (PPG), and PEG-PPG block copolymers; or
(2) a water insoluble composition comprising: (i) particles of a water soluble compound capable of forming channels in the water insoluble composition; or (ii) water insoluble hydrophilic particles which cause swelling of the subcoat when in contact with an aqueous media; and
(D) a pharmaceutically acceptable excipient,
wherein the solid oral dosage form has an onset of release of the compound and/or said pharmaceutically acceptable salt thereof of the core (A) into the ileo-jejunal portion of the small intestine of a mammal after oral ingestion of the dosage form by the mammal; and
wherein the solid oral dosage form is able to release at least 80% by weight of said compound and/or said pharmaceutically acceptable salt thereof in 120 minutes in a dissolution vessel comprising an aqueous solution at a pH of from 6.4 to 7.4.

2. The solid oral dosage form according to claim 1, wherein the solid oral dosage form is able to release less than 10% by weight of said compound and/or said pharmaceutically acceptable salt thereof of the core (A) in 1.5 hours in a dissolution vessel comprising an aqueous solution at a pH of less than 3 or a pH of from 4.5 to 5.5.

3. The solid oral dosage form according to claim 1 or claim 2, wherein the solid oral dosage form releases the compound and/or said pharmaceutically acceptable salt thereof of the core (A) as a bolus in the ileum and/or jejunum of the small intestine of the mammal.

4. The solid oral dosage form according to any one of claims 1 to 3, wherein the compound of the core (A) is chosen from: 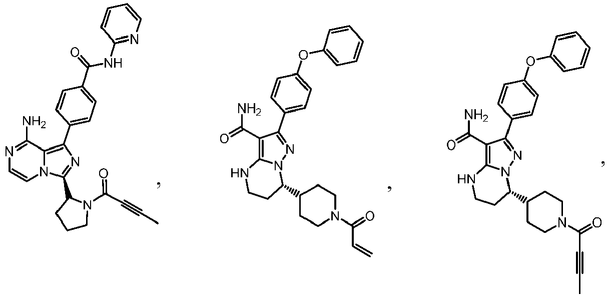 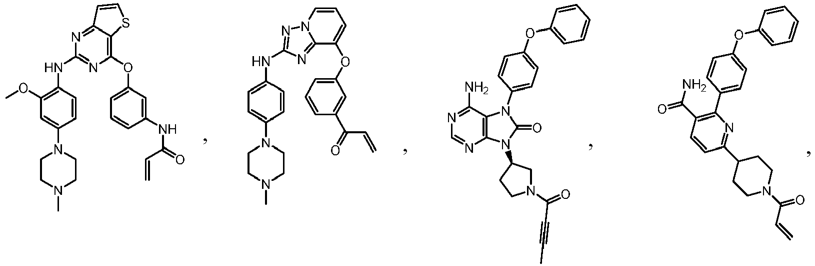 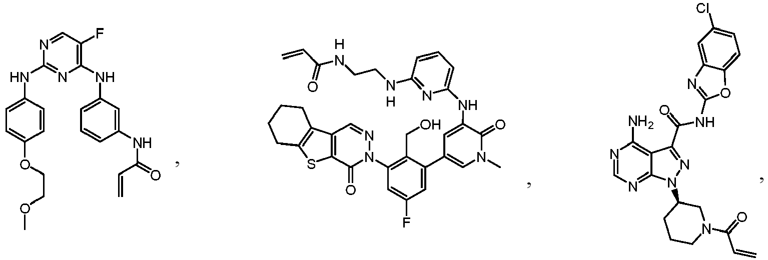 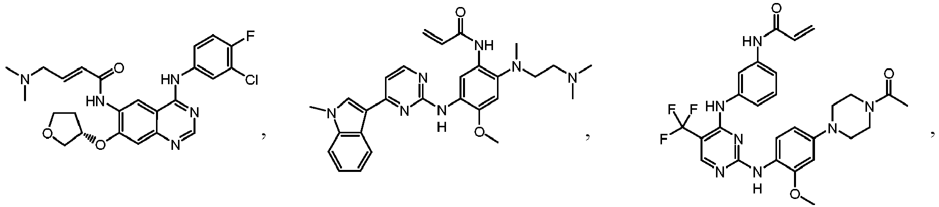 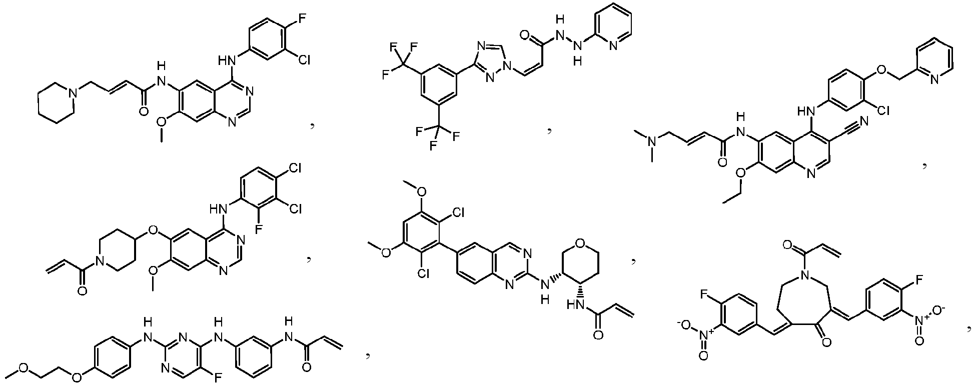 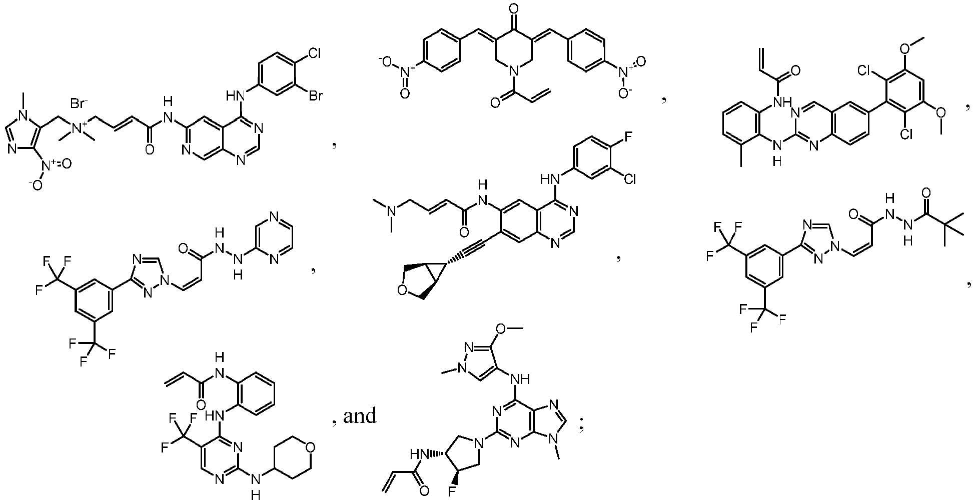 and/or a pharmaceutically acceptable salt thereof.

5. The solid oral dosage form according to any one of claims 1 to 4, wherein the subcoat (C) comprises: (i) particles of a water soluble compound; or (ii) water insoluble hydrophilic particles which cause swelling of the subcoat when in contact with an aqueous media.

6. The solid oral dosage form according to any one of claims 1 to 4, wherein the subcoat (C) is a water insoluble composition comprising particles of a water soluble compound which form channels allowing influx of water into the solid oral dosage form and diffusion of the compound and/or the pharmaceutically acceptable salt thereof into the intestine.

7. The solid oral dosage form according to any one of claims 1 to 4, wherein the subcoat (C) is a water insoluble composition comprising particles of a water soluble compound capable of forming channels that are impermeable to the compound and/or the pharmaceutically acceptable salt thereof but allow entry of water and swelling and rupturing of the subcoat, causing release of the compound and/or the pharmaceutically acceptable salt thereof.

8. The solid oral dosage form according to any one of claims 1 to 7, wherein the aqueous solution is a simulated intestinal fluid at a pH of from 6.4 to 7.4.

9. The solid oral dosage form according to any one of claims 1 to 8, wherein the solid oral dosage form comprises a pharmaceutically acceptable acid salt of the compound.

10. The solid oral dosage form according to any one of claims 1 to 9, wherein the core (A) further comprises:
(a) a pharmaceutically acceptable acid in a quantity sufficient to produce an acidic aqueous solution within the solid oral dosage form prior to the release of the compound and/or the pharmaceutically acceptable salt thereof of the core from the solid oral dosage form; and/or
(b) a surfactant which is present at a concentration above its critical micelle concentration upon disintegration in 50 mL of aqueous media.

11. The solid oral dosage form according to any one of claims 1 to 10, wherein the mean particle size of the compound and/or the pharmaceutically acceptable salt thereof is from 0.3 micron to 100 microns.

12. The solid oral dosage form according to any one of claims 1 to 11, wherein the enteric coating (B):
(a) is from 10% to 150% of the weight of the core;
(b) is from 5 to 500 microns thick;
(c) is selected from polymerized gelatin, shellac, methacrylic acid copolymer type CNF, cellulose butyrate phthalate, cellulose hydrogen phthalate, cellulose proprionate phthalate, polyvinyl acetate phthalate (PVAP), cellulose acetate phthalate (CAP), cellulose acetate trimellitate (CAT), hydroxypropyl methylcellulose phthalate, hydroxypropyl methylcellulose acetate, dioxypropyl methylcellulose succinate, carboxymethyl ethylcellulose (CMEC), hydroxypropyl methylcellulose acetate succinate (HPMCAS), and (meth)acrylic acid polymers and copolymers, wherein said polymers are made from one monomer and said copolymers are made from two or more monomers, wherein said monomers are selected from methyl acrylate, ethyl acrylate, methyl methacrylate, and ethyl methacrylate, preferably wherein the enteric coating comprises a poly(meth)acrylate polymer, optionally wherein the enteric coating is a Eudragit^{®} L or S series, and further optionally wherein the enteric coating is a Eudragit^{®} L100, L12.5, S100, S12.5, or FS 30D;
(d) comprises a cellulose derivative, preferably wherein the cellulose derivative is selected from methylcellulose, cellulose acetate phthalate, hydroxymethyl cellulose phthalate (HPMCP), hydroxypropyl methyl cellulose succinate (HPMCS), and hydroxymethyl cellulose acetate succinate (HPMCAS); and/or
(e) comprises a polyvinyl acetate phthalate (PVAP) polymer.

13. The solid oral dosage form according to any one of claims 1 to 12, wherein the pharmaceutically acceptable excipient (D) is independently selected from binders, surfactants, diluents, buffers, antiadherents, glidants, disintegrants, antioxidants, antifoaming agents, fillers, flavors, colors, lubricants, sorbents, preservatives, plasticizers, and sweeteners.

14. A solid oral dosage form according to any one of claims 1 to 13 for use in a method of treating a disease treatable by inhibition of BTK, in a patient in recognized need thereof, wherein said method comprises administering to the patient a therapeutically effective amount of the compound and/or the pharmaceutically acceptable salt thereof of the core (A) in single or multiple doses.

15. The solid oral dosage form for use according to claim 14, wherein the disease is selected from an autoimmune disease, cancer, and an inflammatory disease, preferably a leukemia or lymphoma, and further optionally wherein the leukemia is selected from chronic lymphocytic leukemia (CLL), small lymphocytic leukemia (SLL), multiple myeloma, mantle cell lymphoma, and B-cell non-Hodgkin lymphoma.

## Patentansprüche

1. Feste orale Dosierungsform, die eine Tablette oder eine Kapsel ist, umfassend:
(A) einen Kern, der eine Verbindung umfasst, ausgewählt aus: 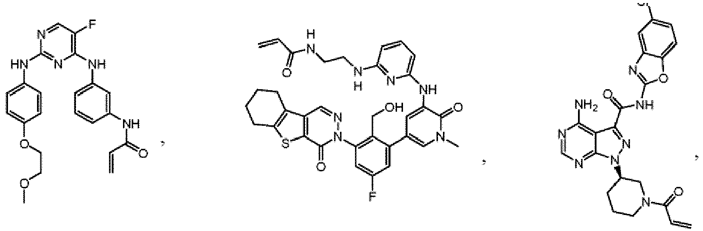 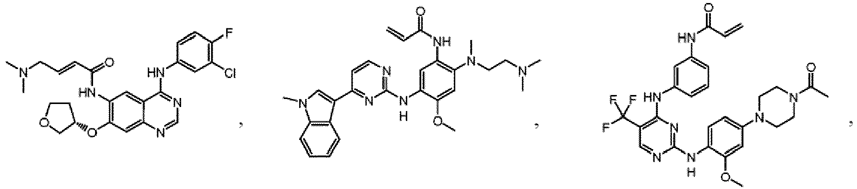 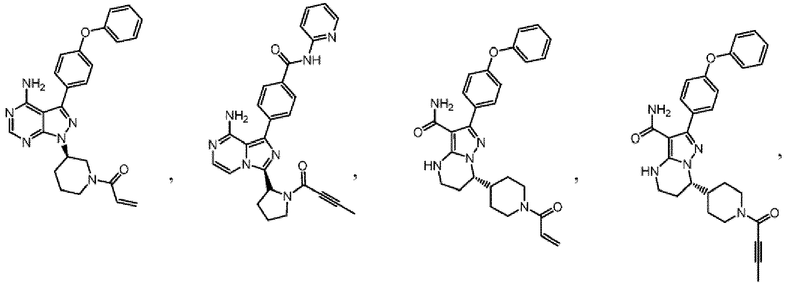 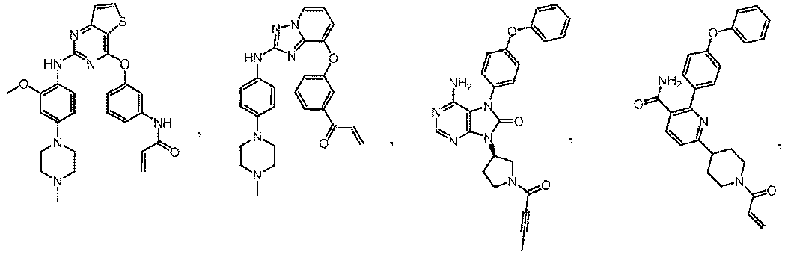 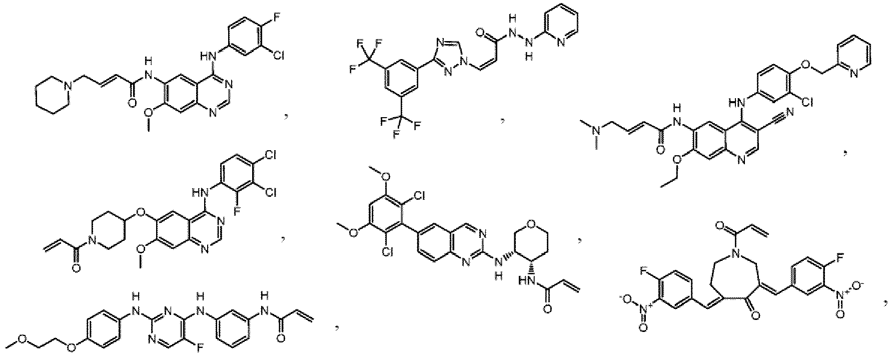 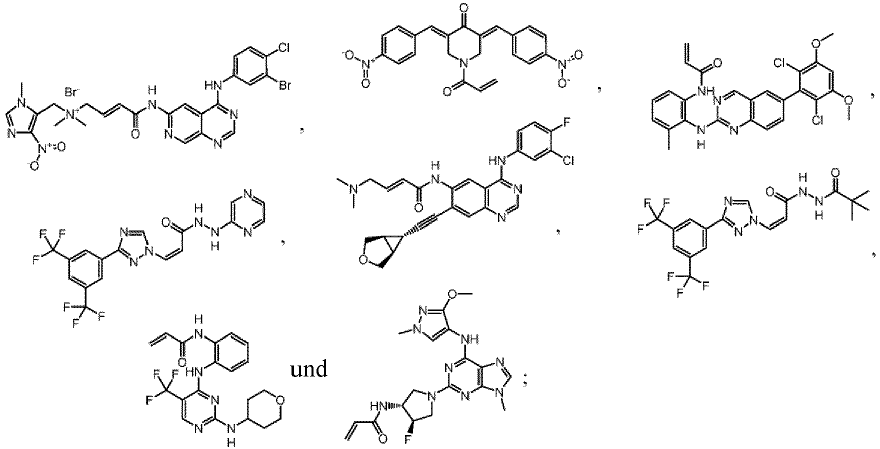 und/oder einem pharmazeutisch unbedenklichen Salz davon;
(B) eine magensaftresistente Beschichtung;
(C) eine Unterschicht zwischen der magensaftresistenten Beschichtung und dem Kern, wobei die Unterschicht ist:
(1) ein wasserlösliches oder hydrophiles erodierbares Polymer, wobei das Polymer ein Polymer mit niedrigem Molekulargewicht ist, ausgewählt aus Hydroxymethylcellulose (HPMC), Hydroxyethylcellulose, Hydroxymethylcellulose, Hydroxypropylcellulose, mikrokristalliner Cellulose, Polyvinylpyrrolidionen, Polysacchariden (oder einem Polysaccharidderivat), Polyvinylalkoholen, Polyethylenglykol (PEG), Polypropylenglykol (PPG) und PEG-PPG-Blockcopolymeren; oder
(2) eine wasserunlösliche Zusammensetzung, umfassend: (i) Teilchen einer wasserlöslichen Verbindung, die in der Lage sind, Kanäle in der wasserunlöslichen Zusammensetzung auszubilden; oder (ii) wasserunlösliche hydrophile Teilchen, die bei Kontakt mit einem wässrigen Medium eine Quellung der Unterschicht verursachen; und
(D) einen pharmazeutisch unbedenklichen Hilfsstoff,
wobei die feste orale Dosierungsform einen Beginn der Freisetzung der Verbindung und/oder des pharmazeutisch unbedenklichen Salzes davon des Kerns (A) in den ileojejunalen Abschnitt des Dünndarms eines Säugetiers nach oraler Aufnahme der Dosierungsform durch das Säugetier aufweist; und
wobei die feste orale Dosierungsform in der Lage ist, wenigstens 80 Gew.-% der Verbindung und/oder des pharmazeutisch unbedenklichen Salzes davon innerhalb von 120 Minuten in einem Auflösungsgefäß freizusetzen, das eine wässrige Lösung mit einem pH-Wert von 6,4 bis 7,4 umfasst.

2. Feste orale Dosierungsform nach Anspruch 1, wobei die feste orale Dosierungsform in der Lage ist, weniger als 10 Gew.-% der Verbindung und/oder des pharmazeutisch unbedenklichen Salzes des Kerns (A) in 1,5 Stunden in einem Auflösungsgefäß freizusetzen, das eine wässrige Lösung mit einem pH-Wert von weniger als 3 oder einem pH-Wert von 4,5 bis 5,5 umfasst.

3. Feste orale Dosierungsform nach Anspruch 1 oder 2, wobei die feste orale Dosierungsform die Verbindung und/oder das pharmazeutisch unbedenkliche Salz davon des Kerns (A) als einen Bolus in dem Ileum und/oder Jejunum des Dünndarms des Säugetiers freisetzt.

4. Feste orale Dosierungsform nach einem der Ansprüche 1 bis 3, wobei die Verbindung des Kerns (A) ausgewählt ist aus: 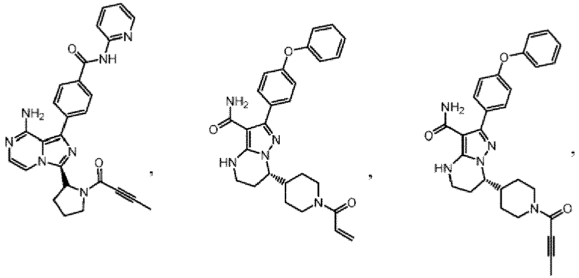 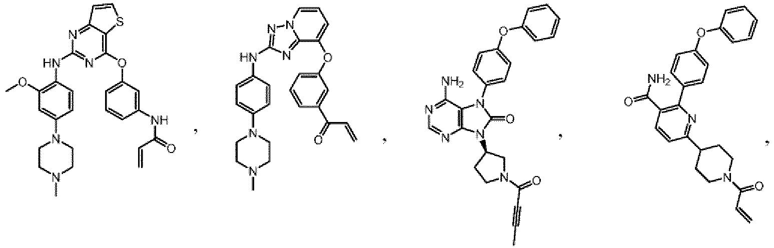 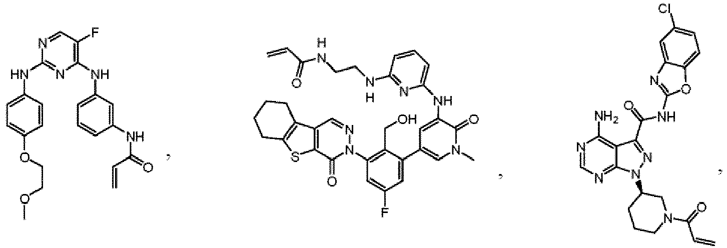 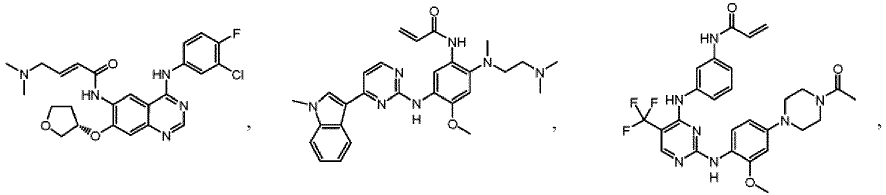 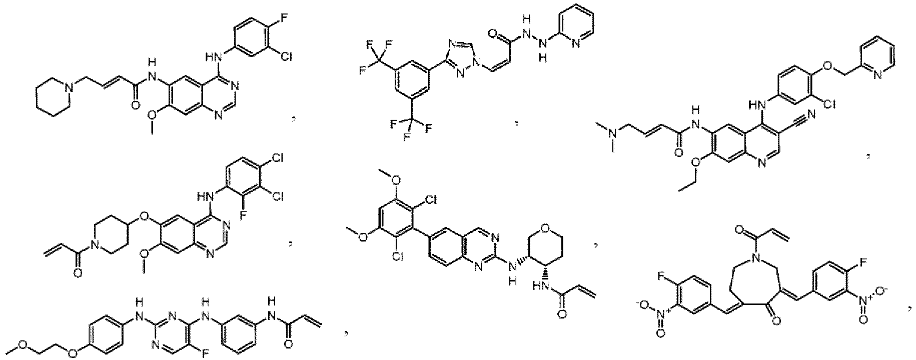 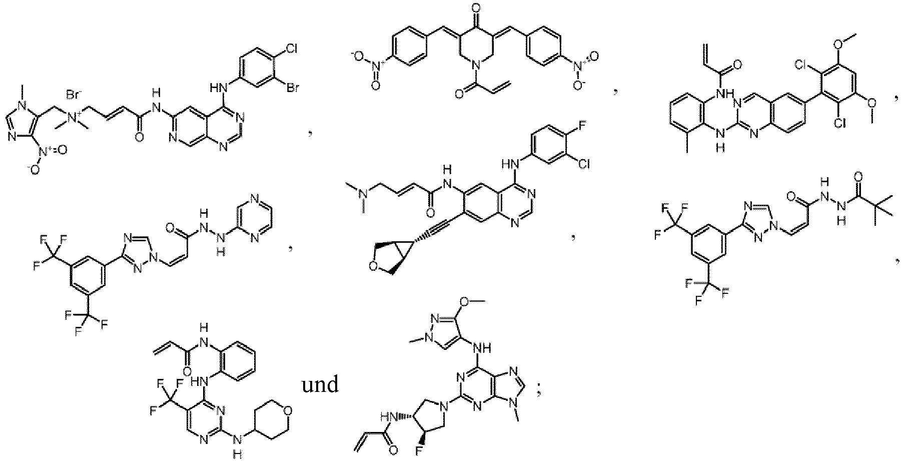 und/oder einem pharmazeutisch unbedenklichen Salz davon.

5. Feste orale Dosierungsform nach einem der Ansprüche 1 bis 4, wobei die Unterschicht (C) umfasst: (i) Teilchen einer wasserlöslichen Verbindung oder (ii) wasserunlösliche hydrophile Teilchen, die bei Kontakt mit einem wässrigen Medium eine Quellung der Unterschicht bewirken.

6. Feste orale Dosierungsform nach einem der Ansprüche 1 bis 4, wobei die Unterschicht (C) eine wasserunlösliche Zusammensetzung ist, die Teilchen einer wasserlöslichen Verbindung umfasst, die Kanäle ausbilden, die den Zufluss von Wasser in die feste orale Dosierungsform und die Diffusion der Verbindung und/oder des pharmazeutisch unbedenklichen Salzes davon in den Darm ermöglichen.

7. Feste orale Dosierungsform nach einem der Ansprüche 1 bis 4, wobei die Unterschicht (C) eine wasserunlösliche Zusammensetzung ist, die Teilchen einer wasserlöslichen Verbindung umfasst, die in der Lage sind, Kanäle auszubilden, die für die Verbindung und/oder das pharmazeutisch unbedenkliche Salz davon undurchlässig sind, aber das Eindringen von Wasser und das Quellen und Aufbrechen der Unterschicht ermöglichen, wodurch eine Freisetzung der Verbindung und/oder des pharmazeutisch unbedenklichen Salzes davon verursacht wird.

8. Feste orale Dosierungsform nach einem der Ansprüche 1 bis 7, wobei die wässrige Lösung eine simulierte Darmflüssigkeit mit einem pH-Wert von 6,4 bis 7,4 ist.

9. Feste orale Dosierungsform nach einem der Ansprüche 1 bis 8, wobei die feste orale Dosierungsform ein pharmazeutisch unbedenkliches Säuresalz der Verbindung umfasst.

10. Feste orale Dosierungsform nach einem der Ansprüche 1 bis 9, wobei der Kern (A) ferner umfasst:
(a) eine pharmazeutisch unbedenkliche Säure in einer Menge, die ausreicht, um eine saure wässrige Lösung innerhalb der festen oralen Dosierungsform zu erzeugen, bevor die Verbindung und/oder das pharmazeutisch unbedenkliche Salz davon aus dem Kern aus der festen oralen Dosierungsform freigesetzt wird; und/oder
(b) ein Tensid, das bei Disintegration in 50 ml wässrigem Medium in einer Konzentration oberhalb seiner kritischen Mizellenkonzentration vorhanden ist.

11. Feste orale Dosierungsform nach einem der Ansprüche 1 bis 10, wobei die mittlere Teilchengröße der Verbindung und/oder des pharmazeutisch unbedenklichen Salzes davon 0,3 Mikrometer bis 100 Mikrometer beträgt.

12. Feste orale Dosierungsform nach einem der Ansprüche 1 bis 11, wobei die magensaftresistente Beschichtung (B):
(a) von 10 % bis 150 % des Gewichts des Kerns ausmacht;
(b) von 5 bis 500 Mikrometer dick ist;
(c) aus polymerisierter Gelatine, Schellack, CNF des Methacrylsäure-Copolymer-Typs, Cellulosebutyratphthalat, Cellulosewasserstoffphthalat, Celluloseproprionatphthalat, Polyvinylacetatphthalat (PVAP), Celluloseacetatphthalat (CAP), Celluloseacetattrimellitat (CAT), Hydroxypropylmethylcellulosephthalat, Hydroxypropylmethylcelluloseacetat, Dioxypropylmethylcellulosesuccinat, Carboxymethylethylcellulose (CMEC), Hydroxypropylmethylcelluloseacetatsuccinat (HPMCAS), und (Meth)acrylsäure-Polymere und -Copolymere ausgewählt ist, wobei die Polymere aus einem Monomer hergestellt sind und die Copolymere aus zwei oder mehr Monomeren hergestellt sind, wobei die Monomere aus Methylacrylat, Ethylacrylat, Methylmethacrylat und Ethylmethacrylat ausgewählt sind, wobei die magensaftresistente Beschichtung vorzugsweise ein Poly(meth)acrylat-Polymer umfasst, wobei die magensaftresistente Beschichtung optional eine Eudragit^{®} L- oder S-Serie ist und wobei die magensaftresistente Beschichtung ferner optional ein Eudragit^{®} L100, L12. 5, S100, S12.5, oder FS 30D ist;
(d) ein Cellulosederivat umfasst, wobei das Cellulosederivat vorzugsweise aus Methylcellulose, Celluloseacetatphthalat, Hydroxymethylcellulosephthalat (HPMCP), Hydroxypropylmethylcellulosesuccinat (HPMCS) und Hydroxymethylcelluloseacetatsuccinat (HPMCAS) ausgewählt ist; und/oder
(e) ein Polyvinylacetatphthalat (PVAP-) Polymer umfasst.

13. Feste orale Dosierungsform nach einem der Ansprüche 1 bis 12, wobei der pharmazeutisch unbedenkliche Hilfstoff (D) unabhängig aus Bindemitteln, Tensiden, Verdünnungsmitteln, Puffern, Haftverhinderern, Gleitmitteln, Sprengmitteln, Antioxidantien, Entschäumern, Füllstoffen, Aromen, Farbstoffen, Schmiermitteln, Sorptionsmitteln, Konservierungsmitteln, Weichmachern und Süßungsmitteln ausgewählt ist.

14. Feste orale Dosierungsform nach einem der Ansprüche 1 bis 13 für die Verwendung in einem Verfahren zum Behandeln einer Erkrankung, die durch Hemmung von BTK behandelbar ist, bei einem Patienten mit anerkanntem Bedarf daran, wobei das Verfahren das Verabreichen einer therapeutisch wirksamen Menge der Verbindung und/oder des pharmazeutisch unbedenklichen Salzes davon des Kerns (A) an den Patienten in einer oder mehreren Dosen umfasst.

15. Feste orale Dosierungsform für die Verwendung nach Anspruch 14, wobei die Erkrankung aus einer Autoimmunerkrankung, Krebs und einer entzündlichen Erkrankung, vorzugsweise einer Leukämie oder einem Lymphom, ausgewählt ist und wobei ferner optional die Leukämie aus chronischer lymphatischer Leukämie (CLL), kleiner lymphatischer Leukämie (SLL), multiplem Myelom, Mantelzell-Lymphom und B-Zell-Non-Hodgkin-Lymphom ausgewählt ist.

## Revendications

1. Forme de dosage orale solide, qui est un comprimé ou une capsule, comprenant :
(A) un noyau comprenant un composé choisi parmi : 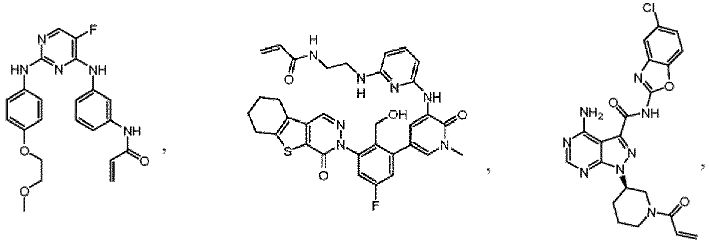 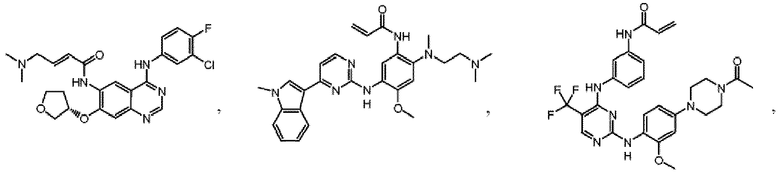 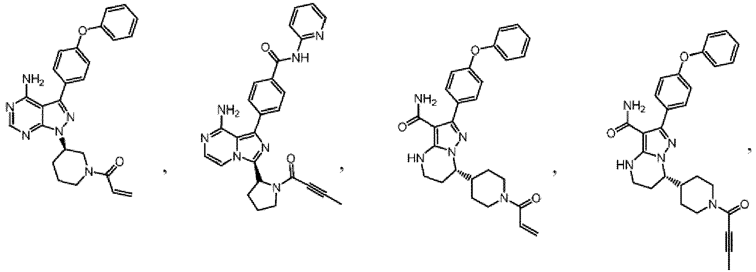 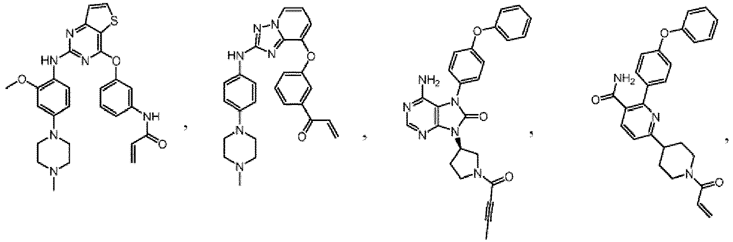 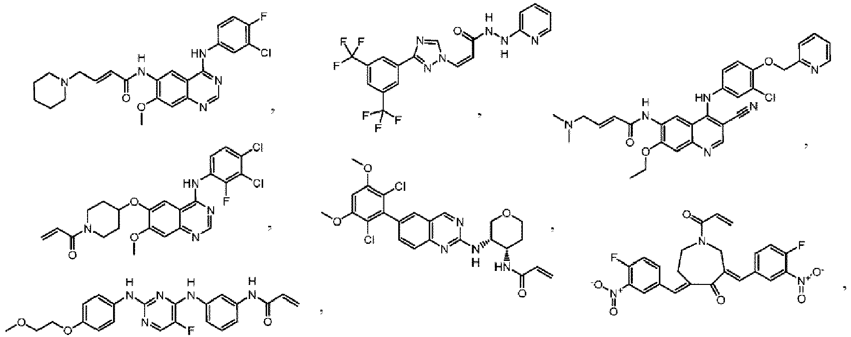 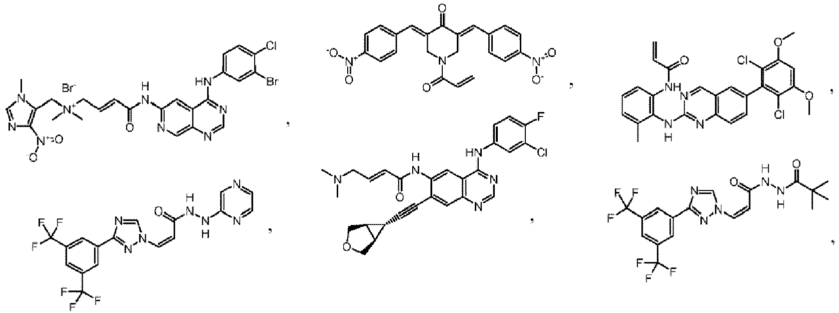 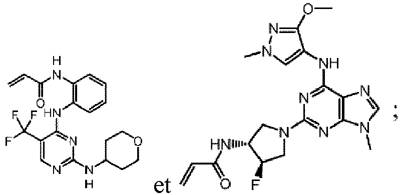 et/ou un sel pharmaceutiquement acceptable de celui-ci ;
(B) un revêtement gastro-résistant ;
(C) un sous-revêtement entre le revêtement gastro-résistant et le noyau, dans laquelle le sous-revêtement est :
(1) un polymère érodable hydrosoluble ou hydrophile, ledit polymère étant un polymère de bas poids moléculaire sélectionné parmi : hydroxyméthylcellulose (HPMC), hydroxyéthylcellulose, hydroxyméthylcellulose, hydroxypropylcellulose, cellulose microcristalline, polyvinylpyrrolidiones, polysaccharides (ou un dérivé de polysaccharide), alcools polyvinyliques, polyéthylène glycol (PEG), polypropylène glycol (PPG), et copolymères blocs PEG-PPG ; ou
(2) une composition hydro-insoluble comprenant : (i) des particules d'un composé hydrosoluble capables de former des canaux dans la composition hydro-insoluble ; ou (ii) des particules hydrophiles hydro-insolubles qui entraînent le gonflement du sous-revêtement lorsqu'il est en contact avec un milieu aqueux ; et
(D) un excipient pharmaceutiquement acceptable,
dans laquelle la forme de dosage orale solide a un commencement de libération du composé, et/ou dudit sel pharmaceutiquement acceptable de celui-ci, du noyau (A) dans la partie iléo-jéjunale de l'intestin grêle d'un mammifère après l'ingestion orale de la forme de dosage par le mammifère ; et
dans laquelle la forme de dosage orale solide est capable de libérer au moins 80 % en poids dudit composé, et/ou dudit sel pharmaceutiquement acceptable de celui-ci, en 120 minutes dans un récipient de dissolution comprenant une solution aqueuse à un pH de 6,4 à 7,4.

2. Forme de dosage orale solide selon la revendication 1, dans laquelle la forme de dosage orale solide est capable de libérer moins de 10 % en poids dudit composé, et/ou dudit sel pharmaceutiquement acceptable de celui-ci, du noyau (A) en 1,5 heures dans un récipient de dissolution comprenant une solution aqueuse à un pH de moins de 3 ou un pH de 4,5 à 5,5.

3. Forme de dosage orale solide selon la revendication 1 ou la revendication 2, dans laquelle la forme de dosage orale solide libère le composé, et/ou ledit sel pharmaceutiquement acceptable de celui-ci, du noyau (A) sous la forme de bolus dans l'iléum et/ou le jéjunum de l'intestin grêle du mammifère.

4. Forme de dosage orale solide selon l'une quelconque des revendications 1 à 3, dans laquelle le composé du noyau (A) est choisi parmi : 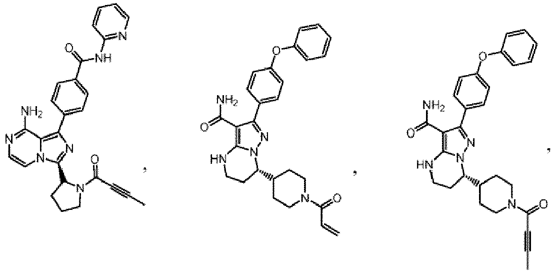 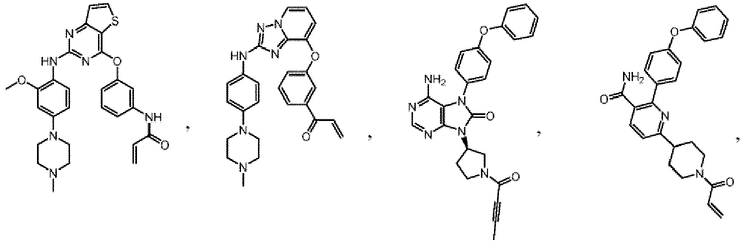 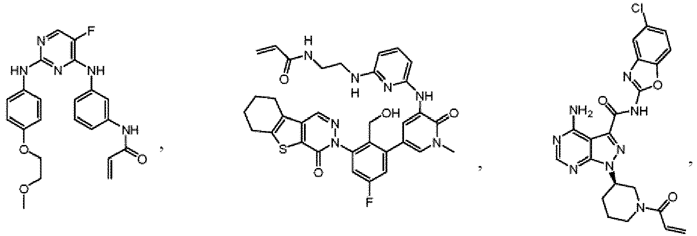 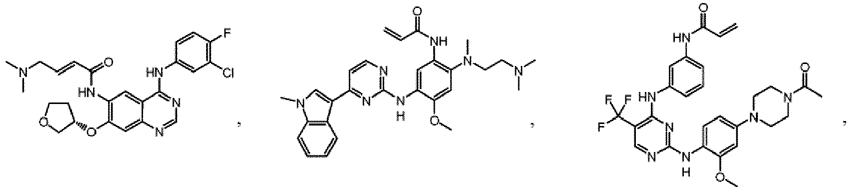 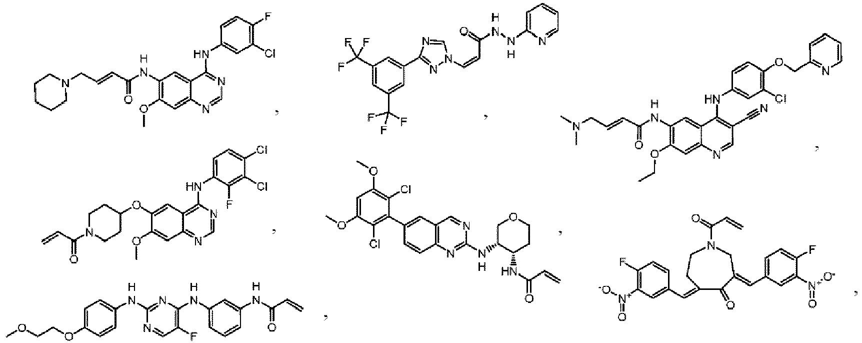 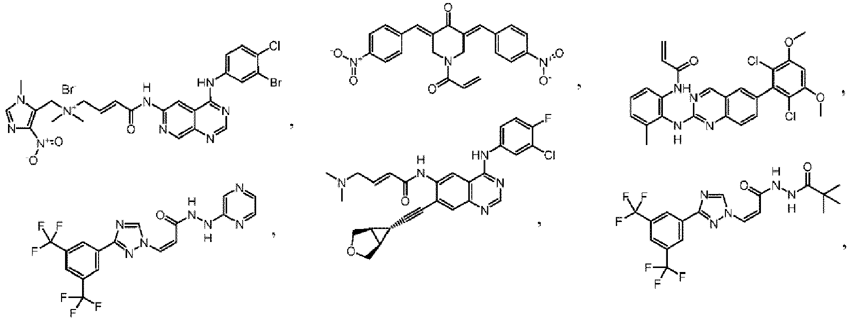 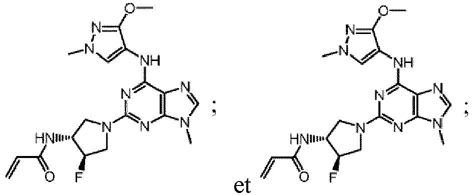 et/ou un sel pharmaceutiquement acceptable de celui-ci.

5. Forme de dosage orale solide selon l'une quelconque des revendications 1 à 4, dans laquelle le sous-revêtement (C) comprend : (i) des particules d'un composé hydrosoluble ; ou (ii) des particules hydrophiles hydro-insolubles qui entraînent le gonflement du sous-revêtement lorsqu'il est en contact avec un milieu aqueux.

6. Forme de dosage orale solide selon l'une quelconque des revendications 1 à 4, dans laquelle le sous-revêtement (C) est une composition hydro-insoluble comprenant des particules d'un composé hydrosoluble qui forment des canaux permettant l'afflux d'eau dans la forme de dosage orale solide et la diffusion du composé, et/ou du sel pharmaceutiquement acceptable de celui-ci, dans l'intestin.

7. Forme de dosage orale solide selon l'une quelconque des revendications 1 à 4, dans laquelle le sous-revêtement (C) est une composition hydro-insoluble comprenant des particules d'un composé hydrosoluble capable de former des canaux qui sont imperméables au composé, et/ou au sel pharmaceutiquement acceptable de celui-ci, mais permettent l'entrée d'eau et le gonflement et la rupture du sous-revêtement, causant la libération du composé, et/ou du sel pharmaceutiquement acceptable de celui-ci.

8. Forme de dosage orale solide selon l'une quelconque des revendications 1 à 7, dans laquelle la solution aqueuse est un fluide intestinal simulé à un pH de 6,4 à 7,4.

9. Forme de dosage orale solide selon l'une quelconque des revendications 1 à 8, dans laquelle la forme de dosage orale solide comprend un sel acide pharmaceutiquement acceptable du composé.

10. Forme de dosage orale solide selon l'une quelconque des revendications 1 à 9, dans laquelle le noyau (A) comprend en outre :
(a) un acide pharmaceutiquement acceptable en une quantité suffisante pour produire une solution aqueuse acide à l'intérieur de la forme de dosage orale solide avant la libération du composé, et/ou du sel pharmaceutiquement acceptable de celui-ci, du noyau à partir de la forme de dosage orale solide ; et/ou
(b) un surfactant qui est présent à une concentration supérieure à sa concentration micellaire critique lors de la désintégration dans 50 mL de milieu aqueux.

11. Forme de dosage orale solide selon l'une quelconque des revendications 1 à 10, dans laquelle la taille moyenne des particules du composé, et/ou du sel pharmaceutiquement acceptable de celui-ci, est de 0,3 micron à 100 microns.

12. Forme de dosage orale solide selon l'une quelconque des revendications 1 à 11, dans laquelle le revêtement gastro-résistant (B) :
(a) est de 10 % à 150 % du poids du noyau ;
(b) fait de 5 à 500 microns d'épaisseur ;
(c) est sélectionné parmi : gélatine polymérisée, gomme laque, copolymère acide méthacrylique de type CNF, phtalate-butyrate de cellulose, phtalate d'hydrogène de cellulose, proprionate-phtalate de cellulose, acétate-phtalate de polyvinyle (PVAP), acétate-phtalate de cellulose (CAP), acétate-triméllitate de cellulose (CAT), phtalate de méthylhydroxypropylcellulose, acétate de méthylhydroxypropylcellulose, succinate de dioxypropylméthylcellulose, carboxyméthyléthylcellulose (CMEC), acétate-succinate de méthylhydroxypropylcellulose (HPMCAS), et polymères et copolymères d'acide (méth)acrylique, dans laquelle lesdits polymères sont faits à partir d'un monomère et lesdits copolymères sont faits de deux, ou plus, monomères, dans laquelle lesdits monomères sont sélectionnés parmi : acrylate de méthyle, acrylate d'éthyle, méthacrylate de méthyle, et méthacrylate d'éthyle, de préférence dans laquelle le revêtement gastro-résistant comprend un polymère poly(méth)acrylate, optionnellement dans laquelle le revêtement gastro-résistant est un Eudragit^{®} série L ou S, et en outre optionnellement dans laquelle le revêtement gastro-résistant est un Eudragit^{®} L100, L12,5, S100, S12,5, ou FS 30D ;
(d) comprend un dérivé de cellulose, de préférence dans laquelle le dérivé de cellulose est sélectionné parmi : méthylcellulose, acétate-phtalate de cellulose, phtalate d'hydroxyméthylcellulose (HPMCP), succinate d'hydroxypropylméthylcellulose (HPMCS), et acétate-succinate d'hydroxyméthylcellulose (HPMCAS) ; et/ou
(e) comprend un polymère acétate-phtalate de polyvinyle (PVAP).

13. Forme de dosage orale solide selon l'une quelconque des revendications 1 à 12, dans laquelle l'excipient pharmaceutiquement acceptable (D) est indépendamment sélectionné parmi : liants, surfactants, diluants, tampons, anti-adhérents, glissants, désintégrants, antioxydants, agents antimousses, charges, arômes, couleurs, lubrifiants, sorbants, conservateurs, plastifiants, et édulcorants.

14. Forme de dosage orale solide selon l'une quelconque des revendications 1 à 13 pour l'utilisation dans un procédé de traitement d'une maladie traitable par inhibition de BTK, chez un patient en ayant un besoin reconnu, dans laquelle ledit procédé comprend l'administration, au patient, d'une quantité thérapeutiquement efficace du composé, et/ou du sel pharmaceutiquement acceptable de celui-ci, du noyau (A) en dose unique ou doses multiples.

15. Forme de dosage orale solide pour l'utilisation selon la revendication 14, dans laquelle la maladie est sélectionnée parmi : une maladie auto-immune, un cancer, et une maladie inflammatoire, de préférence une leucémie ou un lymphome, et en outre optionnellement dans laquelle la leucémie est sélectionnée parmi : leucémie lymphocytaire chronique (CLL), petit lymphome lymphocytaire (SLL), myélome multiple, lymphome à cellules du manteau, et lymphome non-Hodgkinien des cellules B.
